# EUROPEAN PATENT SPECIFICATION

(11) **EP 1 017 855 B1**
(45) Date of publication and mention of the grant of the patent: **03.11.2004**
(21) Application number: 99937170.1
(22) Date of filing: 22.07.1999
(51) Int. Cl.: C12Q 1/68, C12P 19/34

(54) **METHODS OF SYNTHESIZING POLYNUCLEOTIDES BY LIGATION OF MULTIPLE OLIGOMERS**
METHODEN ZUR SYNTHESE VON POLYNUKLEOTIDEN DURCH LIGATION VON MULTIPLEN OLIGOMEREN
SYNTHETISATION DE POLYNUCLEOTIDES PAR LIGATURE D'OLIGOMERES MULTIPLES

(30) Priority: 24.07.1998 US 121887; 02.02.1999 US 241353; 02.02.1999 US 241979; 05.02.1999 US 245984
(43) Date of publication of application: 12.07.2000
(60) Divisional application: 03018758.7
(73) Proprietor: LUMIGEN, INC., Southfield, Michigan 48034 (US)
(72) Inventor: AKHAVAN-TAFTI, Hashem, Howell, MI 48834 (US)
(74) Representative: Jones, Helen Marjorie Meredith
(86) International application number: PCT/US1999/014160
(87) International publication number: WO 2000/005412

(56) References cited:
- EP-A- 0 185 494
- EP-A- 0 359 545
- WO-A-89/11211
- WO-A-89/12696
- WO-A-93/20096
- WO-A-96/35699
- WO-A-97/08344
- WO-A-98/03673
- US-A- 5 763 227

### SEQUENCE LISTING

<110> Akhavan-Tafti, Hashem
<120> METHODS OF SYNTHESIZING POLYNUCLEOTIDES BY LIGATION OF MULTIPLE OLIGOMERS
<130> LUM-4.1-53
<140> 09/121,887
   <141> 1998-07-24
<160> 7
<170> PatentIn Ver. 2.0
<210> 1
   <211> 24
   <212> DNA
   <213> primer
<400> 1
   acttcacttc taatgatgat tatg                                          24
<210> 2
   <211> 24
   <212> DNA
   <213> primer
<400> 2
   ctcttctagt tggcatgctt tgat 24
<210> 3
   <211> 26
   <212> DNA
   <213> primer
<400> 3
   agtggaagaa tttcattctg ttctca 26
<210> 4
   <211> 25
   <212> DNA
   <213> primer
<400> 4
   attaagcaca gtggaagaat ttcat 25
<210> 5
   <211> 65
   <212> DNA
   <213> DNA template
<400> 5
   ttaatggtgc caggcataat ccaggaaaac tgagaacaga atgaaattct tccactgtgc 60
   ttaat 65
<210> 6
   <211> 21
   <212> DNA
   <213> primer
<400> 6
   gaaaccagct tcaaggcact g 21
<210> 7
   <211> 21
   <212> DNA
   <213> primer
<400> 7
   attcagtgcc atgggacata g 21

## Description

### FIELD OF THE INVENTION

The present invention relates generally to methods of synthesizing polynucleotides. More specifically, the invention relates to methods of synthesizing polynucleotides by ligation of a plurality of oligomeric units onto a template-bound primer. The plurality of oligomers can be preselected to contain oligomers which are complementary to the template strand or the oligomers can be supplied as a library and allowed to self select. The synthesis by ligation can proceed unidirectionally or bidirectionally from the primer and can be used to synthesize both strands simultaneously by the use of two primers. Amplification can be performed linearly or exponentially and can be used to copy DNA and RNA. The methods of the invention are useful in a variety of applications, including cloning, preparing labeled polynucleotides for diagnostic use, mutation analysis and screening, gene expression monitoring and sequence analysis.

### BACKGROUND OF THE INVENTION

The enzymatic ligation of pairs of oligonucleotides bound to a target nucleic acid is widely known. It is generally thought that the oligomers must each be of a minimum length to be ligated efficiently. Recent work has shown this minimum length to be about 6-8 bases (C.E. Pritchard and E.M. Southern, Nucl. Acids Res., 25, 3403-3407 (1997)). It is generally thought that ligation of oligonucleotides shorter than about 6 bases is not possible.

Under certain conditions, primer independent ligation can be accomplished using oligomers of at least six bases long. In this manner, PCR primers were prepared *in situ* from concatenated groups of a small number hexamers, heptamers or octamers (T. Kaczorowski and W. Szybalski, Gene, 179, 189-193 (1996); L.E. Kotler, D. Zevin-Sonkin, I.A. Sobolev, A.D. Beskin and L.E. Ulanovsky, Proc. Natl. Acad. Sci. USA, 90, 4241-4245 (1993)). Such ligation in the absence of a primer is undesirable in the present methods and must be avoided. The success in replicating a polynucleotide sequence in a controlled and defined manner rests in knowing the point of origination of the newly synthesized strand.

WO-A-9708344 discloses a ligase-based assay for the identification of mutations in a target sequence. A single stranded target sequence hybridises with, for example, at least three oligonucleotides. The oligonucleotides have sequences which are complimentary to and hybridise with sequential and contiguous portions of the target nucleic acid. The oligonucleotides are subsequently ligated to form a single ligation product.

Nucleic acids can be synthesized from a template, primer and nucleotide triphosphates (NTPs) by the action of a polymerase action. Labels can be incorporated by substituting a percentage of labelled NTPs. The ability to achieve a high degree of label incorporation is limited and the precise spacing of labels is not controllable.

The polymerase chain reaction (PCR) is a method of amplifying the amount of a polynucleotide by the use of a primer complementary to each strand which span the region to be replicated. Nucleic acid synthesis proceeds by extension of each primer with a polymerase and the four dNTPs. Thermal cycling allows multiple copies of the template to be synthesized, approximately doubling the quantity of amplicon in each cycle. A variant termed Ligase Chain Reaction (LCR) involves the ligation of two pairs of oligonucleotides with a ligase enzyme to replicate the sequence of interest (D.Y. Wu and R.B.Wallace, Genomics, 4, 560-569 (1989)). The two oligonucleotides to be ligated constitute the entire length of the strand. Ligation of a large number of small oligomers to a primer to replicate a nucleic acid has not been achieved to the best of Applicant's knowledge.

Methods of providing sequence information using oligonucleotide ligation are disclosed in US 5,750,341 and US 5,770,367 and a publication (S. Dubiley, E. Kirilov, Y. Lysov and A Mirzabekov, Nucl. Acids Res., 25, 2259-2265 (1997)). The reported methods differ fundamentally from those of the present invention in requiring that oligomers be ligated one at a time and the sequence be analyzed after each step. These methods are therefore far more laborious than those of the present invention.

Methods of Labeling Nucleic Acids - Present methods of labeling nucleic acids or oligonucleotides include the tailing method, random primed labeling, nick translation, the labeled branched DNA and end labeling using a labeled primer. Each method suffers disadvantages in certain applications. Use of an end labeled primer extended by PCR with unlabeled bases leads to only one or a few labels per product nucleic acid.

The tailing method incorporates an indeterminate and uncontrolled number of labels by appending a tail of noncomplementary bases onto the nucleic acid of interest. This adds many additional bases, which not only adds expense, but may interfere with hybridization and lead to nonspecific binding. In addition it is not readily applicable to the synthesis of short nucleic acids or oligonucleotides since the length of the tail could exceed the length of the sequence of interest.

The random prime method, applicable to the labeling of long nucleic acids, uses a mixture of primers which are extended by a polymerase with a mixture of labeled and unlabeled bases. The number of bases which can be incorporated is variable and arbitrary in number. A mixture of numerous nucleic acid fragments of varying lengths are produced from both strands. Similarly, nick translation produces a mixture of numerous nucleic acid fragments of varying lengths from both strands. Breaks in both strands of DNA are created and new nucleic acid strands are synthesized from the position of the nick using a mix of labeled and unlabeled bases. Since the position of nicking is arbitrary, label incorporation is not controlled either.

The branched DNA technology has been used in diagnostic tests as a means to attach several labels to a target DNA. The methodology relies on the creation of several branches of synthetic nucleic acid each bound to a probe, followed by hybridization of multiple labeled oligonucleotides to each of the branched amplification multimers. The method requires the costly preparation of many probes and branched DNA and is not generally applicable, especially for the generation of short pieces of highly labeled DNA.

### SUMMARY OF THE INVENTION

It is an object of the present invention to provide methods of synthesizing single or double stranded polynucleotides. It is another object of the present invention to provide methods of synthesizing polynucleotides by ligating a plurality of oligonucleotide 5'-phosphates to a primer hybridized to a template polynucleotide. It is another object of the present invention to provide methods of synthesizing polynucleotides using a library of oligonucleotide 5'-phosphates. It is another object of the present invention to provide methods of synthesizing labeled polynucleotides with a specified position and degree of label incorporation. Another object of the present invention is to provide methods for amplifying the amount of a nucleic acid by primer-directed ligation. Another object of the present invention is to provide methods for the detection of genes and the analysis of gene expression. Yet another object of the present invention is to provide methods for the detection of genetic mutations. Still another object of the present invention is to provide methods for the analysis of the base sequence of a nucleic acid.

The novel method of the invention is defined in claim 1.

### GENERAL DESCRIPTION

It has been discovered that a series of short oligonucleotide-5'-phosphates can be simultaneously ligated onto a template-bound primer in a contiguous manner to produce the complementary strand of a template polynucleotide or nucleic acid. The nucleic acid produced can be either labeled or unlabeled by using either labeled or unlabeled short oligomers. The oligomers in the set each preferably contain the same number of bases. When a sequence to be synthesized is known exactly, a set containing the minimum number of oligomers can be used. The oligomers are ligated in the correct order starting from the primer, to produce the correct sequence. Primer-independent ligation does not occur when using oligonucleotides of length ≤ 5 bases. When the sequence to be synthesized is not known, a library of a large number of the total possible pool of oligomers is used. The latter situation occurs in sequence analysis and mutation screening. Ligations are preferably conducted by means of a ligase enzyme. Known chemical agents for ligating nucleotides and oligonucleotides can be employed as well.

### BRIEF DESCRIPTION OF THE DRAWINGS

Figure 1 schematically depicts the ligation of two oligomers P1 and P2 onto a template-bound primer in the presence of a competing nonligatable complementary oligomer P3.
Figure 2 schematically depicts a method for amplifying the amount of a nucleic acid using template-bound primer directed ligation of multiple oligomers. Synthesis is shown occurring in one direction in each strand, but can also be accomplished bidirectionally as described below.

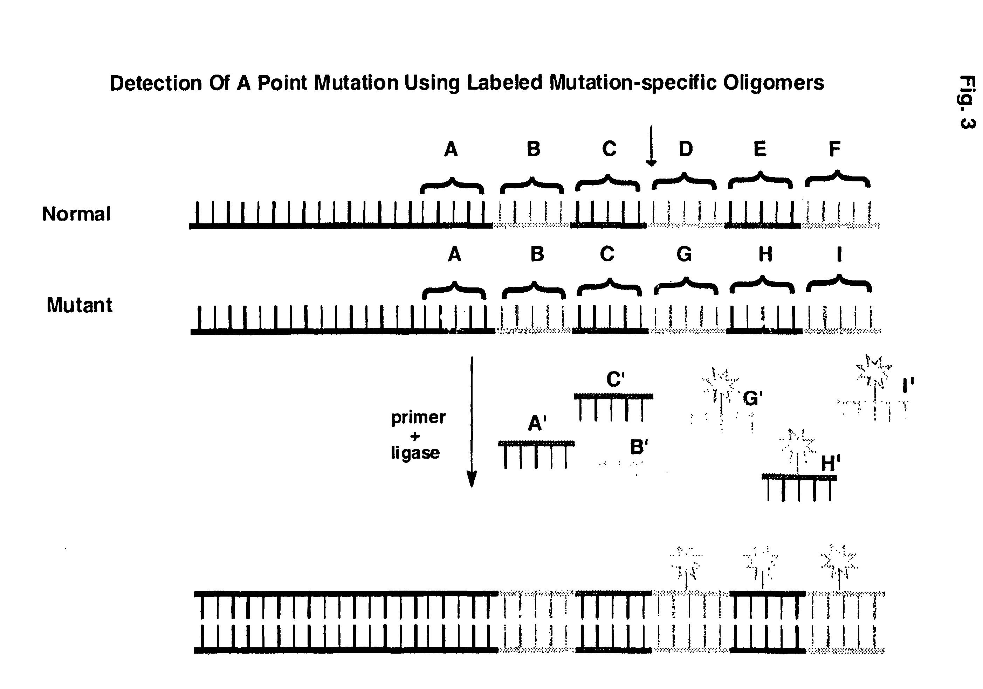

Figure 3 schematically depicts a method for detecting a point mutation in a gene by the ligation of detectably labeled mutation specific oligomers onto a template-bound primer.
Figure 4 schematically depicts a method for detecting two different genotypes of a mutation in a gene by the ligation of different sets of detectably labeled mutation specific or wild-type specific oligomers onto a template-bound primer. The mutation specific oligomers bear a first label while the wild-type specific oligomers bear a second label.
Figure 5 depicts an example of branched DNA or amplification multimers.
Figure 6 depicts an adaptation of branched DNA in which at least the first branch is prepared by ligation of labeled oligomers for providing branch points at regularly spaced intervals.

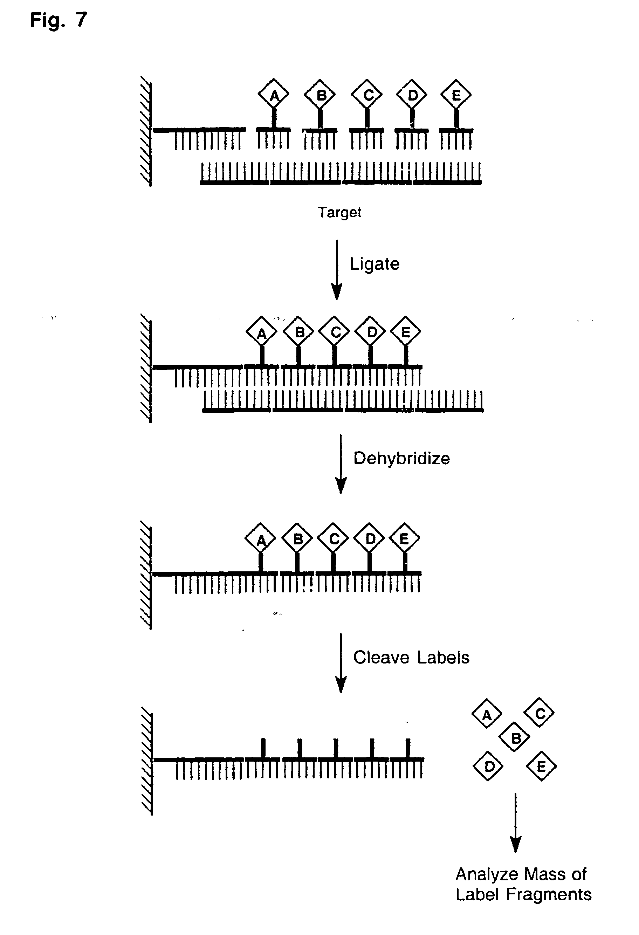

Figure 7 schematically depicts a method for determining the sequence of a nucleic acid by the ligation of unique labeled oligomers onto a template-bound primer, cleaving the labels and analysis of the mass of each unique label.

### DETAILED DESCRIPTION OF THE INVENTION

### Definitions

Oligomer, oligonucleotide - as used herein will refer to a compound containing a phosphodiester internucleotide linkage and a 5'-terminal monophosphate group. The nucleotides can be the normally occurring ribonucleotides A, C, G, and U or deoxyribonucleotides, dA, dC, dG and dT.

Primer or probe/primer - refers to an oligonucleotide used to direct the site of ligation and is required to initiate the ligation process. Primers are of a length sufficient to hybridize stably to the template and represent a unique sequence in the template. Primers will usually be about 15-30 bases in length although longer primers can be used. Labeled primers containing detectable labels or labels which allow solid phase capture are within the scope of the term as used herein. Primer also contemplates contiguously stacked oligomers of at least six bases as is known in the art (T. Kaczorowski and W. Szybalski, Gene, 179, 189-193 (1996)).

Template, test polynucleotide, target are used interchangeably and refer to the nucleic acid whose length is to be replicated.

Sample - A fluid containing or suspected of containing one or more analytes to be assayed. Typical samples which are analyzed by the chemiluminescent reaction method are biological samples including body fluids such as blood, plasma, serum, urine, semen, saliva, cell lysates, tissue extracts and the like. Other types of samples include food samples and environmental samples such as soil or water.

Short oligonucleotide - As used herein, a oligonucleotide 5'-phosphate of at lest two and up to about 10 base length. The bases can be ribonucleotides or deoxyribonucleotides or analogs thereof. The length of a short oligonucleotide useful in a given context can vary within this range and may be less than the whole range. The preferred length varies depending on the particular application.

Specific binding pair - Two substances which exhibit a mutual binding affinity. Examples include antigen-antibody, hapten-antibody or antibody-antibody pairs, complementary oligonucleotides or polynucleotides, avidin-biotin, streptavidin-biotin, hormone-receptor, lectin-carbohydrate, IgG-protein A, nucleic acid-nucleic acid binding protein and nucleic acid-anti-nucleic acid antibody and metal complex-ligand.

A preferred method of ligation uses a ligase such as a DNA ligase. Representative ligases include T4 ligase, T7 ligase, Tth ligase, Taq ligase and *E. coli* DNA ligase. The ligase can be a thermostable ligase, in which case thermal cycling techniques as discussed below are possible. Thermal cycling with a thermostable ligase is useful in methods of amplifying nucleic acids in a manner analogous to the polymerase chain reaction, but using oligomers and a ligase in place of dNTPs and a polymerase. Methods of performing enzymatic ligation reactions are generally described in e.g., Sambrook, et al., *Molecular Cloning: A Laboratory* *Manual*, 2nd Ed., Cold Spring Harbor Laboratory, New York, 1989.

Enzymatic ligation reactions are generally performed in a buffer solution, optionally in the presence of additives to promote hybridization. The buffer has a pH typically in the range of 6 - 9, more usually 7 - 8.5 and preferably in the range 7.5 - 8. Buffers capable of maintaining a pH in this range are suitable. The reaction can be performed over a range of temperatures in the range of 0 to about 50 °C. Optimal temperatures will vary over the range depending on the nature and size of oligonucleotide phosphates to be ligated, the enzyme, presence and amount of additive and can be optimized empirically with reference to the general literature on ligases and by reference to the specific examples below. The length of time for performing the ligation can be as short as a few minutes up to several hours, although it is desirable to conduct the reaction as rapidly as possible. Single stranded DNA binding proteins can be added to oligonucleotide ligation reactions to improve their efficiency. Their effect is due to their relaxation of any secondary structure that is in the template strand thus allowing the complementary oligonucleotides to bind and ligate. E. coli single stranded binding protein (Promega, Madison, WI or Amersham/USB) and T4 Gene 32 protein (Boehringer Mannheim, Indianapolis, IN) can be used. The use of volume excluding agents such as polyethylene glycols (PEG) may be advantageous in promoting ligations. Inclusion of up to 200 mM NaCl may also be useful for promoting ligations. The use of other additives in enzymatic ligations is contemplated and is within the scope of the present methods. Additives include phosphate transfer agents such as ATP, sulfhydryl reagents, including DTT and 2-mercaptoethanol, and divalent cations such as Mg⁺² salts.

Ligation of oligomer 5'-phosphates also comprehends nonenzymatic methods of ligation as well. Chemical reagents which effect the formation of the phosphodiester internucleotide bond are known (CNBr: K.D. James, A.D. Ellington, *Chemistry & Biology*, 4,595,605, (1997); N-cyanoimidazole: T. Li, K.C. Nicalaou, Nature, 369, 218-221 (1994); EDAC: D. Sievers, G. Von Kiedrowski,Nature, 369, 221-224 (1994)). Chemical ligation methods have not been applied to methods of sequence analysis.

Incorporation of mismatched oligomers can occur as in other techniques, especially when the sequence has a high G-C content. The occurrence of mismatches is controllable as is the case with other hybridization methods. Temperature, salt concentration, and additives can all be employed in art-recognized manners to control the stringency of the hybridization process. Since the effect of a mismatch on a small oligomer should be proportionately greater than on a larger one, discrimination of improper sequences may show improvement over other ligation techniques.

Another embodiment uses a library of possible sequences to achieve the ligation of a series of short oligomers of length n bases to synthesize a complementary nucleic acid. The library contains many more possible combinations of the n bases (n-mers) than are required to form the product nucleic acid. When n=5, for example, there are 4⁵ or 1024 possible 5-mers which contain the four naturally occurring bases A, C, G, T and U. The library can contain all 4ⁿ possible oligomers or less than the full set, but should contain at least a substantial proportion (> 50%, and preferably > 75%, most preferably >90%) of the possible oligomers.

Known methods of synthesizing polynucleotides, by polymerase extensions with dntps or ligation of preformed oligonucleotides, function by providing only a small number of different reactants for incorporation into the product molecule. Known ligation-based methods usually preselect the one oligonucleotide with the correct sequence. Polymerase extension methods supply the four individual bases for incorporation. The present methods differ fundamentally in providing a large number of potential reactants into the reaction mixture. Moreover, a significant number of the short oligonucleotides have a sequence appropriate for hybridization to the target but, if hybridized, would block or prematurely terminate the ligation process.

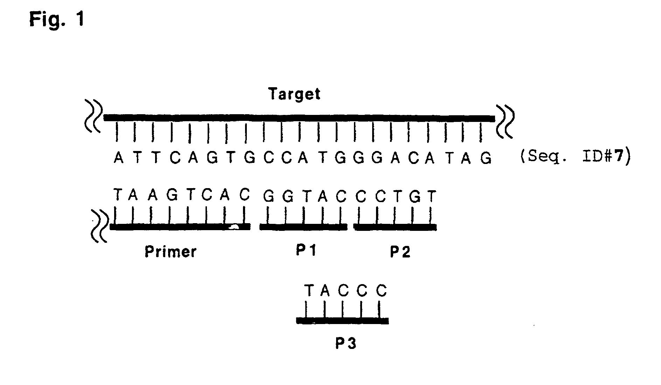

As seen in Figure 1, oligomer P3 is complementary to a portion of the target sequence, but, if hybridized, would block ligation of P1 and P2 to the primer. Surprisingly, the presence of complementary oligomers which can not be ligated onto the primer does not interfere with or prevent the successful ligation of the desired oligomers to the template-bound primer.

The library also will contain a majority of oligonucleotide sequences which are not complementary to the target or only partially complementary. This excess of oligonucleotides, in effect, competes with the correct sequences for recognition and ligation. Nevertheless, ligation of short oligonucleotides in the correct order does occur effectively in spite of the statistical unlikelihood. The ability to faithfully replicate a nucleic acid by successive ligation of many short oligonucleotides in one step is unexpected and greatly simplifies the process compared to others known in the art.

The length of oligonucleotides to use in the present methods is governed by the interplay of several competing factors. Larger oligomers will hybridize more strongly under a given set of conditions (salt concentration, temperature) and can therefore hybridize at a higher temperature. As the length of the oligonucleotide increases, the number of discrete compounds required to assemble the complete library of all possible n-mers increases by a factor of 4 for each unit increase of n.

| Length of Oligomer | Total # of Sequences |
|---|---|
| 1 | 4 |
| 2 | 16 |
| 3 | 64 |
| 4 | 256 |
| 5 | 1024 |
| 6 | 4096 |
| 7 | 16,384 |
| 8 | 65,536 |
| 9 | 262,144 |
| 10 | 1,048,576 |

Shorter oligomers require less compounds to construct the entire library, but become more difficult, e.g. lower temperature, to hybridize and ligate as their length decreases. This, in turn, translates to greater stringency at a given temperature. Still another factor is the ability of the oligonucleotide to hybridize and initiate extension at a site not associated with the primer. Primer-independent hybridization has been demonstrated to occur, under the right conditions, with oligonucleotides as small as 6 bases. Ligation of 2 or more contiguous hexamers to produce e.g., a dodecamer or octadecamer, then effectively produces a new primer. If this happens, the ability to control the starting point for polynucleotide synthesis is compromised. On the other hand, the probability of finding multiple occurrences of a given sequence in a nucleic acid of hundreds of bases increases substantially as shorter oligonucleotides are used. In applications involving sequence determination, it is desirable to avoid or minimize the occurrence of duplicate sequence elements. The selection of the optimum length oligonucleotide to use is a compromise among these conflicting effects. The optimum length will be different in different end uses.

In practice it may not be necessary to use the full library of oligonucleotides of length n. When the number of oligonucleotides required to produce the given sequence is small compared to the total number of oligonucleotides in the library, partial libraries can be used and still maintain a high probability that all of the required oligonucleotides will be present. In some instances it may be desirable to exclude certain sequence oligonucleotides which hybridize too weakly or strongly.

It is not necessary in the present methods, except as explicitly noted below, that each component of the set of oligonucleotide 5'-phosphates used in a given method be of the same number of bases. It can be advantageous in some embodiments to use a combination of oligomers of two or more different lengths, such as pentamers and hexamers, in order to avoid the occurrence of duplicate oligomers. It is often preferred that the oligonucleotides used in a given method be of the same number of bases. This number is preferably 2 to 6, more preferably 5.

In another embodiment, template-directed ligation of a plurality of a set of short oligonucleotides of the same length onto a primer can be performed in a manner which controls the endpoint of the ligation by the use of nonextendable oligomers. A nonextendable oligomer can contain the same or a different number of bases as the other oligomers in the set. The nonextendable oligomer contains a 5'-phosphate so that it can be ligated, but lacks the 3'-OH group. It could, for example, have a dideoxy base at the 3'-end of the oligomer so that there is no 3'-OH for ligation. Another type of nonextendable oligomer contains a blocked 3'-OH group for example where the hydroxyl group is blocked with a methyl group or a phosphate group, to prevent subsequent ligation. Modifications to the terminal base which prevent ligation are another possible type of nonextendable oligomer. The nonextendable oligomer can be labeled or unlabeled, depending on the need. A preferred embodiment is to use oligomers containing a dideoxy base at the 3'-terminus.

Another aspect of the present invention is a method for synthesizing a nucleic acid by ligation of a plurality of oligonucleotide 5'-phosphates onto a template-bound primer in both the 5'→3' and 3'→5' direction at the same time. This process can be performed, for example, by providing a 5'-phosphate group on the primer. Ligation can occur simultaneously from both termini of the primer as long as the appropriate ligatable oligomers are provided. The point of termination of synthesis in either or both directions can be controlled by the use of nonextendable oligomers or by excluding selected oligomers. A nonextendable oligomer for terminating synthesis in the 3'→5' direction would not have the 5'-phosphate group.

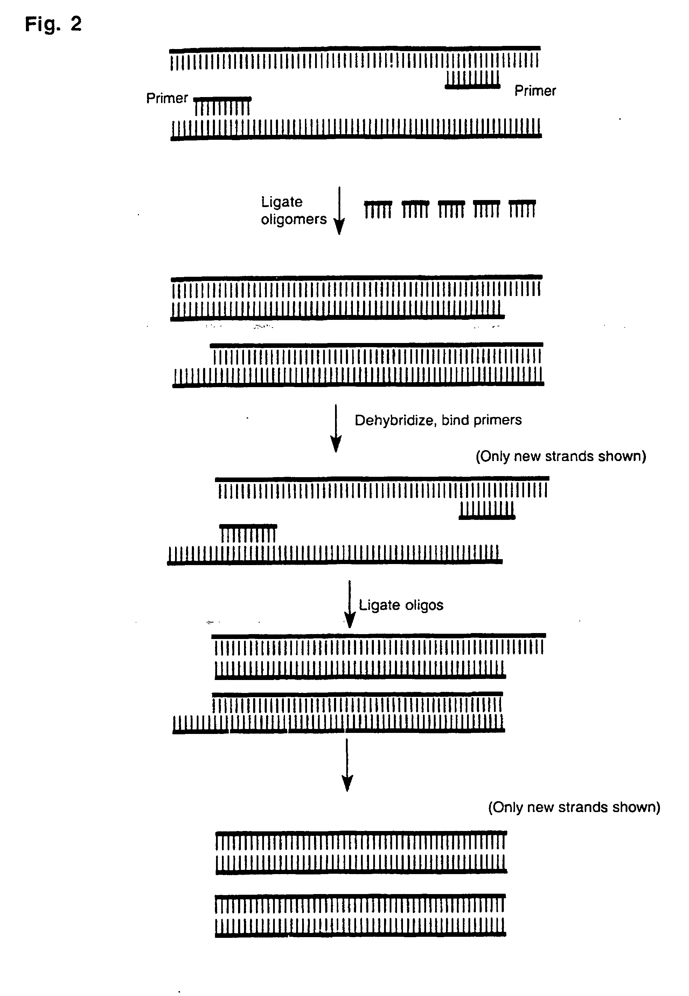

The template-directed ligation of a plurality of a set of short oligonucleotides onto a primer can be used in a method of amplifying the quantity of a target DNA. Accordingly, another aspect of the invention comprises a method of amplifying a target nucleic acid using a ligase, two primers and a set of short oligonucleotides where the probes are complementary to regions on opposing strands spanning the region of the target to be amplified. At a minimum, the oligomer set supplied for reaction must contain those oligomers required to extend both primers on their respective strands as far as the position corresponding to the 5' end of the other primer. Additional oligomers can be included, for example as would occur when using the entire library of oligomers instead of preselecting the set of oligomers. The process, shown schematically in Figure 2, is distinct from the polymerase chain reaction, PCR, but using a library of oligomers and a ligase instead of the four deoxyribonucleotides and a polymerase. Each cycle of annealing, ligase extension and dehybridization results in a two-fold amplification of the target sequence. Since heating is generally required for separating the newly synthesized duplex nucleic acid, additional ligase may need to be added in subsequent rounds of ligation-extension. Alternatively, the process can be performed with a thermostable ligase. Thermal cycling can then be performed without replacing the ligase every cycle.

In accordance with the above description there is provided a method for amplifying the amount of a portion of a double stranded nucleic acid having a first strand and a second strand as defined in claim 2.

In a preferred embodiment of an amplification process, the set of oligomers is preselected to contain only those oligomers necessary to replicate the two strands, i.e. those oligomers occurring on the two strands in the region spanned by the two primers. In another preferred embodiment, nonextendable oligomers are used for the terminal positions of each strand. These two terminating oligomers, by definition, have a base sequence complementary to the first group of bases of the length of the oligomer at the 5' end of each primer.

Amplification methods in accordance with the present invention can be achieved by synthesis of each strand in both the 5'→3' and 3'→5' direction at the same time. This bidirectional amplification process can be performed, for example, by providing a 5'-phosphate group on the primer. Ligation can occur simultaneously from both termini of each primer as long as the appropriate ligatable oligomers are provided. The point of termination of synthesis in either or both directions can be controlled by the use of nonextendable oligomers or by excluding selected oligomers as described above.

As is the case with other uses of the present oligomer ligation method of synthesizing nucleic acid, either labeled or unlabeled oligomers can be used. The set of oligomers used can be the entire library, a substantial portion of the library or a preselected subset if the sequence to be amplified is known in advance.

In another aspect, the method of synthesizing specific nucleic acid sequences by ligating oligomers onto target bound primers can be used in diagnostic applications. Specific sequences characteristic of the target of interest can be detected using labeled oligomers in the method of synthesizing the new strand. When the base sequence of the target nucleic acid region is known, the corresponding oligomers needed to complete this sequence are used, at least some of which should carry a detectable label. Such methods have use in many areas of nucleic acid diagnostics, including detection of infectious agents such as *C. trachomatis* and *N. gonorrhoeae, P. carinii, M. tuberculosis*, detection of food borne pathogens such as *Salmonella* and *E. coli*, methods of detecting the expression of genes in high throughput screening assays, methods of detecting genetic abnormalities, forensic testing of DNA samples from suspected criminals, identity matching of human remains and paternity testing a specific sequence and differentiating it from other related sequences.

In the area of genetic abnormality testing, one application is a method for the detection of genetic mutations. The mutations can be a point mutation (a and β-Thalassemia), a single base substitution (Sickle Cell Anemia), a deletion (Cystic Fibrosis ΔF₅₀₈, Tay-Sachs), an insertion, a duplication, a transposition of bases or a combination of the above. Labeled oligomers are selected for ligation to a probe/primer such that the resulting extended primer is a labeled mutation-specific polynucleotide.

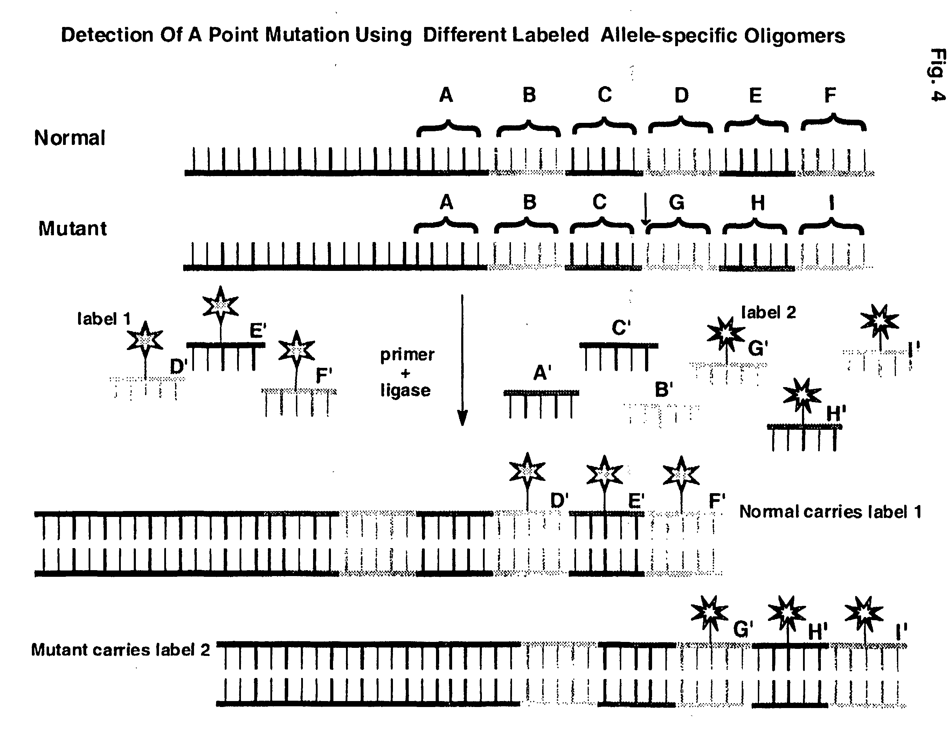

The methods of the present invention can be used to provide a method for the differentiation of heterozygotes from homozygotes for such a genetic condition. Since two copies of a chromosome containing a DNA sequence of interest are present in a sample, the method of synthesizing labeled complementary DNA provides a means for distinguishing heterozygotes from either homozygote. A set of oligomers carrying a first label which, when ligated, produce a portion of a strand complementary to the normal sequence is provided for ligation. Another set of oligomers carrying a second label produces a portion of a strand complementary to the mutant sequence upon ligation (Figure 4). Ligating the sets of oligomers to a probe hybridized to target DNA in the sample creates a polynucleotide complementary to the sample genotype. The three genotypes are resolved by the determining which labels are present in the newly synthesized DNA. Homozygous DNA will contain one label or the other; heterozygous DNA will contain both.

When the sequence to be synthesized is not known, a library of a large number of the total possible pool of oligomers is used. The latter situation occurs in sequence analysis and mutation screening. When used in conjunction with the methods of ascertaining the base sequence of a newly synthesized polynucleotide described in detail below, numerous mutations of a particular gene can be analyzed and identified simultaneously. The ability to test for multiple mutations in a gene would enable screening for genetic diseases such as cystic fibrosis for which more than 500 mutations have been identified.

In yet another aspect, the primer is immobilised.

After ligation the oligonucleotide 5'-monophosphates which are not ligated are removed and the captured probe-test nucleic acid hybrid having an extended double stranded region is denatured to remove the test nucleic acid from the solid support and produce the immobilized single stranded nucleic acid.

The method controls the point of origin and is not limited by the size of oligomer to be ligated. When the sequence to be transcribed is exactly known, the oligomers can be pre-selected to reduce cost and complexity.

Another aspect of the invention is a method of synthesizing multiply labeled nucleic acid where the extent of label incorporation is controlled and provides a high density of labeling. When using an immobilized primer to serve the dual purpose of capture probe and primer the captured hybrid is contacted with a plurality of labeled, oligonucleotide 5'-monophosphates, following ligation the labeled oligonucleotide 5'-monophosphates which are not ligated are removed; and the captured probe-test nucleic acid hybrid having an extended double stranded region is denatured to remove the test nucleic acid from the solid support and produce an immobilized labeled single stranded nucleic acid containing a plurality of labels.

The primer can alternatively be a nonimmobilized primer for the purposes of synthesizing a multiply labeled nucleic acid. This embodiment utilises a plurality of labeled oligonucleotide 5'-monophosphates and after ligation the labeled oligonucleotide 5'-monophosphates which are not ligated are removed.

The method can further comprise the step of separating the extended primer strand from the template nucleic acid strand if desired. The label borne on each oligonucleotide 5'-monophosphate can be different or all can be the same label. Alternatively, a limited number of different labels, e.g. 2-5 labels, can be employed. The choice of labels used will be governed by the final application.

The present methods, in contrast to other methods of labeling nucleic acids described in the Background section, can prepare virtually any length nucleic acid, but would probably be most useful for products of at least about 50 bases. Shorter products would have less labels attached. One of the main advantages is that the degree and position of label attachment is precisely controlled. For example, pentamers bearing one label each lead to product in which every fifth base is labeled, providing a label density of 20 %. Still higher densities can be achieved with shorter oligomers or with pentamers bearing two or more labels each.

The ability to controllably label at these high densities will be particularly advantageous in diagnostic tests where detection sensitivity is paramount. Higher label densities should translate to improved limits of detection. Controlled labeling will contribute to improved assay precision. The labels can be virtually detectable species, including radioisotopes, chemiluminescent labels and fluorescent labels, colorimetric labels detected on the basis of absorption of light, specific binding molecules including antigens and antibodies, binding proteins such as streptavidin and haptens such as biotin and digoxigenin. In addition, when the label is a small hapten, the detectable label can be a species such as an enzyme which is bound to the nucleic acid via an enzyme-anti-hapten conjugate. In the latter regard, the use of pentamer ligation to produce labeled nucleic acid provides still another advantage. The bulky enzyme labels would be attached at every fifth base, which places them at nearly 180° angles along the double helix from the nearest neighboring label. Consideration of the internucleotide separation and molecular diameters of enzymes, reveals that even relatively large globular proteins can be accommodated at this labeling density without severe steric congestion.

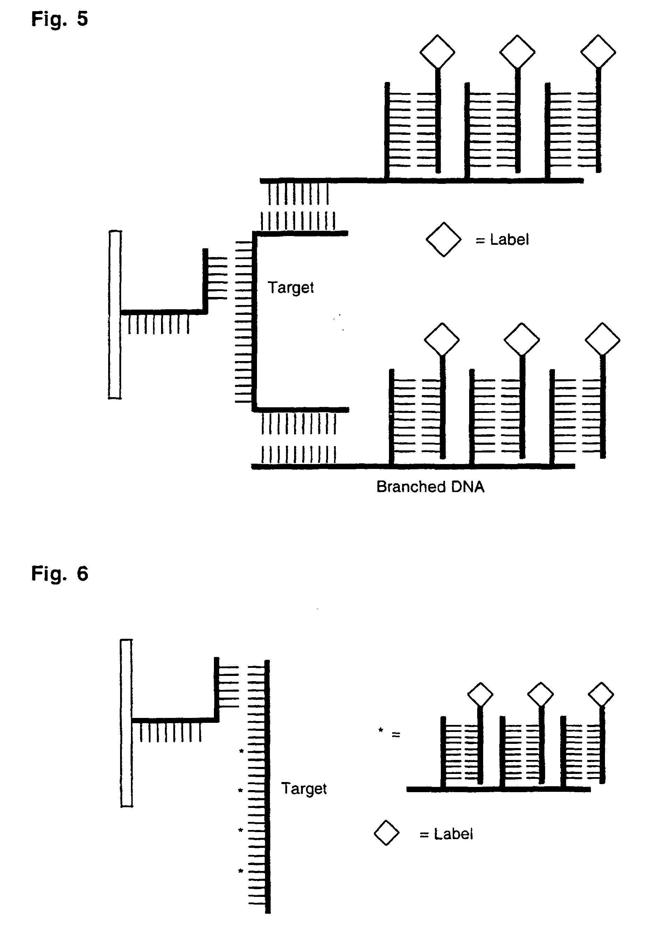

Still higher label densities can be achieved by adopting the branching label principle in conjunction with the incorporation of regular labeled oligomers as depicted in Figures 5 and 6. In practice, some or all of the oligomers would constitute a "handle" such as a hapten or short recognition sequence which is used to bind to a branched amplification multimer.

Alternately, the arms of the branches could be prepared by the ligation of labeled short oligomers, so that each of the multiple arms carries detectable labels. Synthesis of densely labeled nucleic acids by ligation of labeled oligomers can be adapted to other types of branching DNA technology such as the DNA dendrimers (Polyprobe, Philadelphia).

The oligonucleotide 5'-phosphates used in the above-disclosed methods of synthesis, amplification, preparing labeled polynucleotides or immobilized polynucleotides are preferably relatively short. In these applications, it is not necessary to use a substantial fraction of the total library of oligomers of a given length in order to be able to synthesize the desired nucleic acid of known sequence. The size of the oligomers can take any convenient value, typically from 2 to about 20 bases. When high density labeling is desired, it is preferred that the oligomers contain less than about 10 bases and preferably from about 4 to about 8 bases.

In another aspect of the invention, methods are provided for determining the sequence (sequencing) of an unknown single stranded nucleic acid. The method can be applied to RNA, ssDNA and denatured dsDNA sequences of suitable lengths provided that at least a portion of the sequence is known. The latter restriction is necessary in order that a capture probe/primer may be designed.

The capture probe/primer is immobilized or capable of being immobilized onto a solid support such as a bead, tube, filter, membrane microtiter plate or chip. The capture probe should be of sufficient base length to guarantee efficient hybridization and represent a unique partial sequence on the test nucleic acid. These conditions will generally be satisfied with a length of at least 10 bases and preferably at least 15 bases. The capture probe can be immobilized onto the solid support in any art-recognized way. A commonly used means is to provide a biotin label for binding to a streptavidin-coated support. Streptavidin-coated beads and microtiter plates are commercially available.

The oligonucleotide 5'-phosphates used in sequence analysis determinations performed in accordance with the methods disclosed herein are preferably relatively short. It is necessary to use a substantial fraction of all possible oligomers of a given length in order to be able to synthesize long stretches of nucleic acid of unknown sequence. In order to keep the total library size manageable, it is desirable to limit the size of the oligomer to less than about 8 bases. It is more preferred that the oligomers contain 5 or 6 bases. A further requirement in embodiments involving sequence analysis is that all oligonucleotide 5'-phosphates be of the same number of bases.

Cleavable labels can be any molecular fragment capable of being controllably released from the extended probe/primer. Preferred labels are small organic molecules of molecular mass less than about 50,000 amu. It is desirable that the labels all be of one structural type, having a common functional group so that all are cleavable by a common means. One means for effecting cleavage is by thermolysis of a thermally labile group. A preferred thermally labile group for use in cleavable labels is a 1,2-dioxetane. It is well known that 1,2-dioxetanes undergo a thermal fragmentation of the dioxetane ring to produce two carbonyl fragments. Dioxetane-labeled oligomers can be prepared which release a carbonyl compound when heated by tethering a dioxetane group to a ribonucleotide or deoxyribonucleotide.

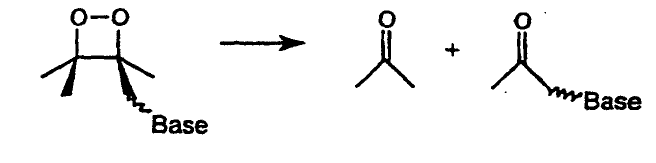

A library of oligomers would comprise the set of all possible sequences of n bases, each covalently attached to a unique dioxetane moiety. For convenience of synthesis, the linking functionality connecting the dioxetane ring group to the oligomer should be common to all members of the family of labeled oligomers. Substituents on the dioxetane ring at the carbon which is cleaved will vary among the members of the set of compounds.

Other thermally cleavable functional groups such as noncyclic peroxides are known and can be used. The temperature required for thermolysis must be low enough so that oligonucleotide fragmentation does not occur.

The means of cleaving the cleavable label is not limited to thermal cleavage. Any means of controllably releasing the label from the extended probe/primer can be employed. Other means include, without limitation, enzymatic reactions, chemical reactions including nucleophilic displacements such as fluoride-induced silyl ether cleavage, basic or acidic hydrolytic fragmentations such as ester hydrolysis or vinyl ether hydrolysis, photochemical fragmentations, reductive cleavage such as metal-induced reductive cleavage of a disulfide or peroxide, oxidative cleavage of alkenes or diols.

An exemplary enzymatic reaction for label cleavage utilizes enzymatically triggerable dioxetanes as labels. Enzymatic deprotection of a protected phenolic substituent triggers cleavage of the dioxetane ring into two carbonyl compounds as depicted above. The reaction can be performed at room temperature and the rate of cleavage controlled by the amount and nature of the triggering enzyme and the characteristics of the reaction solution, e.g. pH. Numerous triggerable dioxetane structures are well known in the art and have been the subject of numerous patents. The spiroadamantyl-stabilized dioxetanes disclosed in U.S. 5,707,559 are one example, others containing alkyl or cycloalkyl substituents as disclosed in U.S. 5,578,253 would also be suitable. A linking substituent from the aforementioned spiroadamantyl, alkyl or cycloalkyl groups would be required to attach the dioxetane label to the oligomer. Linkable dioxetanes are disclosed in U.S. 5,770,743.

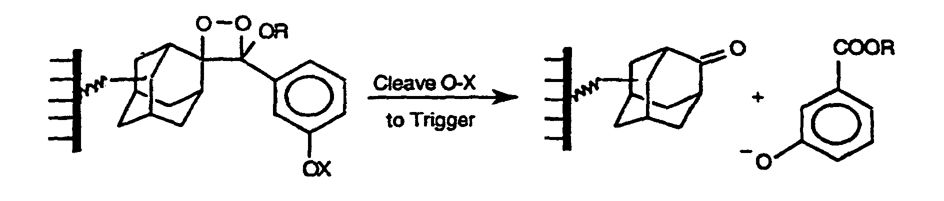

Chemical methods of cleaving triggerable dioxetanes are also well known and would be similarly useful in the methods of the invention. In the example above, X can be a trialkylsilyl group and the triggering agent fluoride. Other triggering agent/cleavable group pairs are described in, for example, the aforementioned 5,707,559, 5,578,253 and 5,770,743 patents.

There are several ways in which the label can be detected. Each method or type of label constitutes a different embodiment of the invention. In one embodiment, the label is a fluorescent molecule such as the fluorescers FAM, JOE, ROX and TAMRA commonly used in automated dideoxy sequencing. Numerous methods of labeling nucleotides and oligonucleotides are known in the art and include direct attachment of label (Haugland, *Handbook of Fluorescent Probes and Research Chemicals*, (Molecular Probes, Eugene, OR), 1992). Labeling can also be accomplished by indirect means where, for example, where a universal linker such as biotin is provided as the primary label and a fluorescer-labeled binding partner for biotin provides the label.

In another embodiment, the label is a chemiluminescent compound and the quantity of label is detected by the light intensity produced by triggering the generation of chemiluminescence from the label. Several types of chemiluminescent compounds are known and can be used as labels. Representative examples include acridinium esters and sulfonamides, luminol or isoluminol derivatives, and dioxetanes (R. Handley, H. Akhavan-Tafti, A.P. Schaap, J. Clin. Ligand Assay, 20(4) 302-312 (1997)). A preferred chemiluminescent label is an acridan phosphate compound as disclosed in Applicant's co-pending application WO-A-9966328 The latter compounds are used advantageously because of their stability, high chemiluminescence quantum efficiency, ease of.conjugation and ability to be triggered under a wide range of conditions, including in electrophoresis gels. Bioluminescent and electrochemiluminescent compounds are considered within the scope of detectable chemiluminescent labels.

In another embodiment, the label is a chromogenic compound and the quantity of label is detected by light absorbance. Another label type is a radioisotope such as ³²P and ³⁵ S whose presence can be detected using scintillation counting or x-ray imaging. The label can also be an enzyme such as alkaline phosphatase, β-galactosidase, luciferase and horseradish peroxidase. The quantity of enzyme is determined by measuring the action of the enzyme on a fluorogenic, chromogenic or chemiluminogenic substrate.

Another aspect of the invention comprises a method of detecting a target nucleic acid by detecting a labeled extended nucleic acid which is complementary to the target, the method being a simpler alternative than traditional Southern and northern blotting. Preparation of the labeled extended complementary nucleic acid is performed by ligation of a plurality of labeled short oligomers onto a probe/primer which is hybridized to the target. Extension is followed by denaturing electrophoretic separation and detection of the labeled species. The presence of the labeled extended primer is indicative of the presence of the target since ligation only takes place when the primer is hybridized to the target. It is preferred that the label is detectable in the gel. Suitable labels include acridan alkenes as described in WO-A-9966328 which can be detected by chemiluminescence, and fluorescers which are readily detectable in gels. In this embodiment, no blotting is performed. If the label is such that detection in the gel is not feasible, then blotting onto membrane is performed and then detection of the label is performed on the membrane. In no case is hybridization on the membrane, antibody binding, enzyme-conjugate binding, substrate addition or other commonly used methods necessary.

In an alternate embodiment of this method, the labeled extended complementary nucleic acid remains hybridized to the target and after electrophoresis, the band detected at the appropriate molecular weight in the manner described above. This mode may be desirable when accurate molecular weight information is needed. In this method it is more convenient to provide substantially the full library of possible oligomer 5'-phosphates of n bases when the target is much longer than the probe/primer. In cases where the target sequence is known and where its length makes it more practical, it may be preferred to preselect the subset of oligonucleotide 5'-phosphates.

Yet another embodiment comprises providing a suitable fluorescent donor as a label on a probe/primer and a suitable fluorescent acceptor as a label on the oligomers. It is not necessary to label each oligomer. Ligation is performed on hybridized primer to form an extended primer bearing a fluorescent donor and one or more fluorescent acceptor labels. Under suitable conditions, i.e. when the donor and acceptor possess sufficient spectral overlap for energy transfer to be feasible and the spatial separation between donor and acceptors are within the Förster distance, energy transfer between fluorescers can occur within the extended primer. Irradiation of the extended primer at a wavelength absorbed by the fluorescent donor on the primer results in fluorescence from the acceptor on the extended portion. This method can therefore serve as the basis for a homogeneous assay for detecting a target nucleic acid since the presence of target is required to permit the ligation to occur and thereby bring the fluorophores within energy transfer distance.

Another method for detecting a target nucleic acid based on the ligation of a plurality of labeled oligomers comprises using a fluorescent intercalating dye as a label. It is known that certain dyes become fluorescent when intercalated within the double helix of double stranded nucleic acids. An example is the widely used compound ethidium bromide.

As an optional step, agarose can be added to the reaction to enhance fluorescence. In the absence of target, ligation does not occur, so the detection of fluorescence is evidence of the presence of the target and additionally is evidence that the primer was sufficiently complementary to the target to hybridize. The collection of oligomers can be the full library of all possible sequences, or a subset containing preselected members if the target sequence is known.

The fraction of labeled oligomers to use can be selected empirically with regard to the desired degree of detection sensitivity by using a range of different label densities. It may be desirable, depending on the size of the oligonucleotide 5'-phosphates, to limit the fraction of labeled oligomers to avoid self quenching of fluorescence.

Synthesis of oligomers - Oligonucleotides are readily synthesized using standard methods of synthesis well known to those of skill in the art including, e.g:, phosphoramidate chemistry. Phosphorylation of oligonucleotides is performed using a polynucleotide kinase and ATP or by chemical methods of phosphorylation as described in (L.A. Slotin, Synthesis, 737-752 (1977); T. Horn, M. Urdea, Tetrahedon Lett., 27, 4705-4708 (1986)). A kit is commercially available for carrying out 5'-phosphorylation (Phosphate-ON, Clontech, Palo Alto, CA).

Methods for the automated synthesis of oligonucleotides are well known in the art and in common commercial use. A common method uses a solid support of immobilization and automated reagent handling to add nucleotides sequentially. All addition, blocking and deblocking steps are under computer control. Such instruments are available from several commercial suppliers such as Applied Biosystems, CA (Model 392 and 394). Automated instruments for transfer of liquid reagents and samples can be performed under computer control using laboratory robots such as are commercially available (Perkin-Elmer, model 800 Catalyst, Beckman Instruments Biomek). Newer techniques for the high speed synthesis or synthesis of large numbers of oligonucleotides utilize photolithographic techniques or ink jet technology for the rapid and precise delivery of reagents and reactants.

Still a further aspect of the invention comprises annealing a primer oligonucleotide 5'-phosphate to a single-stranded template and, in the manner disclosed above, ligating a library of oligomers to extend the primer from both ends to duplicate the template strand. The method is useful in a method to render a single stranded template double stranded in order to clone it. This would find utility in methods for isolating related genes or gene families.

In an exemplary method, a primer oligonucleotide is hybridized to a template strand in the presence of a library of all possible combinations of pentamers, a DNA ligase, and an appropriate reaction buffer. Pentamers that are complementary to the template strand, and in exact register with the 5' and 3' ends of the primer oligonucleotide, anneal and are sequentially ligated by the action of the DNA ligase. The template strand thereby becomes substantially copied or rendered double-stranded. This procedure can be used to detect target templates in a mixture of nucleic acid strands and to prepare double-stranded nucleic acids for cloning using cloning vectors and techniques known in the art.

### EXAMPLES

### Example 1. General Procedure

The template used in this experiment was a PCR amplified product (200 bp) of exon 10 region of the cystic fibrosis transmembrane regulator (CFTR) gene. The PCR-amplified DNA of the CFTR gene was purified either by running it through a column (Qiaquick PCR purification kit, Qiagen, Santa Clarita, CA) or by ethanol precipitation. The DNA was resuspended in distilled water at a concentration of approximately 0.5 µg/µL. Pentamers bearing a 5'-phosphate group and primers were obtained commercially (Oligos Etc., Wilsonville, OR).

The primer and pentamers were designed to be complimentary to either the sense or the antisense strand of the template used. The length of the primer used in these experiments ranged from 21 to 26 nucleotides. The pentamers were designed in such a way that the first pentamer anneals to the template immediately adjacent to the 3' end of the primer. The subsequent primers line up contiguously starting at the 3' end of the first pentamer. Hybridization of the primer and pentamers to the template followed by ligation by T4 DNA ligase results in back to back ligation at the 5'-3' junctions. To enable the detection of the ligated primer-pentamer products, biotin-dUTP labeled pentamers (at the internal dTTP position) were used.

Hybridization of the primer and pentamers to the template and their ligation to each other was accomplished in a 3-step process. First, the template-primer-pentamer mix was heated to 94 °C and kept for 5 min to allow the denaturation of the double stranded template. The mix was cooled to 60 °C or 65 °C,depending on the size and base composition of the primer, to anneal the primer to the template for 2 min. Finally, the reaction tubes were cooled to 16 °C. After about 2 min at 16 °C, ligation buffer (66 mM Tris HCl, pH 7.6, 6.6 mM MgCl₂, 10mM DTT, 66 µM ATP, Amersham) and T4 DNA ligase, 1 U (Amersham, 1:10 dilution) were added and ligated at 16 °C for 2 hours. The ligation reaction was stopped by adding 1/10th volume of loading dye (0.01% xylene cyanol and 0.01% bromophenol blue, and 0.01 M EDTA in deionized formamide).

The ligation reactions were electrophoresed on a denaturing polyacrylamide gel along with biotin-labeled oligonucleotide size markers. The DNA was capillary transferred to a nylon membrane, bound with anti-biotin antibody-HRP conjugate, and detected by reacting with Lumigen PS-3 (a chemiluminescent HRP substrate) and exposing to an x-ray film. The size of the ligated product varies depending on the number of pentamers ligated to the primer.

### Example 2. Determining Optimal Concentrations of Template, Primer and Pentamers.

Template: A 200 bp PCR product of CFTR exon 10 (See the attachment for the template DNA sequence) was obtained by PCR amplification using a set of sense (5' ACTTCACTTCTAATGATGATTATG 3') (Seq. ID#1) and an antisense (5' CTCTTCTAGTTGGCATGCTTTGAT 3') (Seq. ID#2) primers.

A 26 base oligonucleotide complementary to the sense strand of the template DNA was designed as a primer (5' AGTGGAAGAATTTCATTCTGTTCTCA 3') (Seq. ID#3).

Pentamers: Six 5-base long oligonucleotide 5'-phosphates complementary to the sense strand immediately adjacent the 3' end of the primer were prepared. The 5' end of the first pentamer aligns immediately next to the 3' end of the primer, the 5' end of the second pentamer aligns immediately next the 3' end of the first pentamer and so on. To facilitate ligation, the 5' end of each pentamer was phosphorylated. To enable the detection of the ligation products, pentamers 1 and 3 were labeled with biotin-dUTP at the central dTTP position and the last pentamer was labeled with biotin at the 3' end. The pentamers were as follows:
Pentamer 1: 5' PO₄-GTTTT 3'
Pentamer 2: 5' PO₄-CCU*GG 3' U* = U-Biotin
Pentamer 3: 5' PO₄-ATTAT 3'
Pentamer 4: 5' PO₄-GCCU*G 3'
Pentamer 5: 5' PO₄-GCACC 3'
Pentamer 6: 5' PO₄-ATTAA 3'-Biotin.

Ligations were performed using T4 DNA ligase and ligation buffer (Amersham), according to the ligation conditions described in Example 1. The ligations were performed in a volume of 20 µL. The amount of template was kept constant at about 1 µg per reaction. The amount of primer was varied from 100 ng to 1 pg between reactions. The amount of each pentamer was varied from 2 ng to 0.2 pg in each reaction. The reaction with 1 µg of template, 100 ng of primer, and 2 ng of each pentamer contained approximately equimolar concentrations of the template, primer and pentamers. The primer and pentamers were varied systematically to determine the lowest amount of detectable ligation product.

The ligation reactions were electrophoresed, capillary transferred to a nylon membrane, bound with anti-biotin antibody-HRP conjugate, and detected with Lumigen PS-3 as described in Example 1. A full-length primer-pentamer ligation product of expected size (56 bp) was detected in the ligation reaction containing 100 ng of primer and 20 ng of each pentamer (0.6 µM). Lower concentrations of the primer and pentamers in the ligation reaction yielded very low amount or no detectable ligation product under these conditions.

### Example 3. Ligations with varying number of pentamers.

To show that the pentamers are sequentially ligated to the primer starting from the first pentamer (immediately downstream from the primer), ligations were performed using the template, primer and pentamers of Example 2 by incrementing the number of pentamers in each reaction. All the reactions contained equimolar concentrations (0.6 µM) of the template, primer and each pentamer. The ligations were performed as described above and the products detected by binding with anti-biotin-HRP antibody and reacting with Lumigen PS-3 substrate.

As expected, the size of the ligation product increased incrementally by five bases with the addition of each pentamer starting from the first pentamer and so on. There was no ligation product in the absence of the first pentamer and with the rest of the pentamers in the reaction demonstrating the requirement of the primer and specificity of the pentamers for the ligation to occur. In one ligation reaction containing the first four pentamers, there were two bands of the ligated product one of which was the expected size and the other was the size expected when all five pentamers are present in the reaction. Comparing the sequences of the pentamers revealed a single base difference between the third and fifth pentamers. The third pentamer appears to hybridize at the fifth pentamer position when the fourth pentamer is present in the reaction.

| Reaction | Pentamers Used | Product Length |
|---|---|---|
| 1 | 2, 3, 4, 5, 6 | 26 (primer) |
| 2 | 1, 2 | 36 |
| 3 | 1, 2, 3 | 41 |
| 4 | 1, 2, 3, 4 | 46 |
| 5 | 1, 2, 3, 4, 5 | 51, 56 |
| 6 | 1, 2, 3, 4, 5, 6 | 56 |

In reaction 1, the product, which consisted of the primer alone, was detected by virtue of a label on the primer.

### Example 4. Competition from "Out of Register" Pentamer Set.

This example demonstrates that the ligation of a set of pentamers to a primer in a contiguous chain starting from the 3' end of the primer is not affected by the presence of a second set of pentamers which are also complementary to the template and also align contiguously, but begin at a position 'one-base-out' from the 3' end of the primer. Both the correct pentamer sets (1-8) and one-base-out pentamer sets (la-5a) were included in the reaction along with the template and primer during the denaturing, annealing at 60 °C and ligation steps. The ligation reactions contained equimolar (0.6 µM) concen-trations of template, primer and each of the pentamers.

The target region of the template comprises the sequence: 3' TAA TTC GTG TCA CCT TCT TAA AGT AAG ACA AGA GTC AAA AGG ACC TAA TAC GGA CCG TGG TAA TT 5' (Seq. ID#5).

| Reaction | Pentamers Used | Product Length |
|---|---|---|
| 1 | 1, 1a-6a | 31 (primer + 5) |
| 2 | 1, 2, 1a-6a | 36 |
| 3 | 1-3, 1a-6a | 41 |
| 4 | 1-4, 1a-6a | 46 |
| 5 | 1-5, 1a-6a | 51 |
| 6 | 1-6, 1a-6a | 56 |
| 7 | 1-7, 1a-6a | 61 |
| 8 | 1-8, 1a-6a | 66 |
| 9 | 2-8, 1a | 26 (primer) |
| 10 | 1-8, 1a-6a(no primer) | none |

Even in the presence of a set of six competing pentamers, the ligation products formed were the result of ligation of the "correct" pentamers being ligated to the primer. Reaction 10 confirmed that the presence of primer is required for ligation to occur. The one-base-out pentamers did not appear to interfere with the ligation of the correct pentamers.

### Example 5. Competition from Labeled "Out of Register" Pentamer.

The experiment as Example 4 but pentamer 1a was biotinylated. This afforded the opportunity to directly observe the formation of any ligation products from the set of pentamers 1a-6a. No ligation products were observed from the set of one-base-out pentamers.

### Example 6. Ligation Experiments Using JH Downstream Template.

Primer-directed pentamer ligation products were also obtained using as the template a 700 bp DNA downstream of immunoglobulin heavy chain joining region (JH) cloned into a plasmid vector. The JH downstream region was amplified by PCR, cloned into a plasmid vector which was then digested with Eco RI to obtain a sufficient amount of the template DNA. The restriction digest was separated on an agarose gel, and the DNA band of interest extracted using a gel extraction kit (Qiagen). The DNA was resuspended in distilled water at a concentration of approximately 0.5 µg/µl.

The primer and pentamers used with this template are shown below:

Ligations were performed with 500 ng of template, 100 ng of primer, and 20 ng of each pentamer in each 20 µL ligation reaction. The number of pentamers was incrementally increased in each successive ligation reaction to show that the size of the ligation product grew in 5 base increments with each addition of a pentamer.

After performing the ligation reactions according to the general method of Example 1, there was a 5-base incremental increase in the size of the ligation product with each addition of successive pentamers. There were two bands in the ligation reaction containing the first four pentamers, the upper band being more intense than the lower band. The size of the upper band was the same as when all five pentamers were used for the ligation. This is probably because there is sequence similarity between the third and fifth pentamers, so the third pentamer was ligated also at the fifth pentamer position.

### Example 7. Ligation at Various Temperatures.

Ligations using the template, primer and the first four pentamers of Example 6 were performed at 30 °C, 37 °C, 40 °C and 45 °C to examine the effect of ligation temperature on mismatch discrimination. The template, primer and pentamer concentrations were the same as in the previous experiment. Parallel reactions were performed and the relative amount of the four pentamer and five pentamer extension products assessed. At 30 °C ligation temperature, the correct size and the non-specific ligation products were of the same intensity. More of the correct size ligation product was detected at ligation temperatures of 37 °C and 40 °C. At 45 °C, the amount of correct size ligation product detected was diminished.

### Example 8. Ligation Using Octamers.

Ligations of octamers with 5' phosphate and an internal biotin label to a 23mer primer specific to pUC18 plasmid template were performed using T4 DNA ligase (Amersham), Taq DNA ligase (New England Biolabs), and Ampligase™ (Epicenter Technologies, Madison, WI), an NAD-dependent thermostable ligase, each using their respective ligation buffers. In these experiments, 100 to 800 ng of EcoRI linearized plasmid template was used. The concentrations of the primer and the octamers were 12 pmols per reaction.

In experiments with T4 DNA ligase and Taq DNA ligase, first the template, primer and pentamer mix was heated at 94 °C for 5 min, cooled to 60 °C for 2 min for annealing the primer to the template, and further lowered to 16 °C (for T4 DNA ligase) or 45 °C (for Taq DNA ligase) for 2 min. Then the respective ligation buffers and enzymes (1 U /reaction) were added and ligated for 2 h.

For ligation with Ampligase, the ligation mix containing the template, primer and octamers (same as above) was heated at 94 °C for 5 min, then cycling temperatures of 94 °C for 5 min and 45 °C for 5 min (35 cycles) were used for ligation. In another experiment using the Ampligase, a short PCR product of pUC18 (100 bp) was used as template and the conditions of ligation were 94 °C for 1 min, 55 °C for 1 min, and 15 °C for 5 min for a total of 20 cycles.

The ligation products were electrophoresed on an 8% PAGE-Urea gel, semi-dry blotted (Hoefer TE90 blotter) onto a positively charged nylon membrane, UV cross-linked, incubated with anti-biotin antibody conjugated to HRP and detected with Lumigen PS-3 substrate.

With T4 DNA ligase and Ampligase using the linearized pUC18 plasmid, a ligation product of the expected size was detected. However, under the conditions used, Ampligase appeared to be much less efficient than T4 DNA ligase as was evident from the amounts of ligation product detected. Taq DNA ligase reaction did not yield a detectable ligation product. Using the short pCR product of pUC18, Ampligase ligation produced more ligation product than using the full-length pUC18.

### Example 9. Bidirectional Ligation

In this experiment, a 5'-phosphorylated primer was ligated at both ends to a set of pentamers with 5'-phosphate using T4 DNA ligase. A CFTR PCR product template, primer and pentamer mix was heated at 94 °C for 5 min, the primer annealed at 65 °C for 5 min, and the pentamers ligated at 16 °C for 2 h. The ligation buffer and the enzyme were added after the reaction mix was at 16 °C for 2 min. The reaction mixture was electrophoresed, blotted and detected as in Example 8. The pentamers were ligated to the primer in both the 5' and 3' directions from the primer. Product bands of the size observed could only be produced by ligation of each of the pentamers in the set. The signals of ligation products resulting from the ligation of pentamers at the 5' end of the primer were less intense than those ligated at the 3' end of the primer. This could be either due to differences in the ligation efficiency in two directions, or in the labelling with biotin of the two pentamer sets.

### Example 10. Isothermal Ligation at Various Temperatures.

Ligation of a set of 5-phosphorylated octamers onto a template-bound primer was performed at a constant temperature without prior heating and reannealing of the primer to the template. All the components of the ligation reaction - the template, primer, octamers, ligation buffer and ligase were added at room temperature and ligated at either 25 °C, 30 °C, 37 °C or 45 °C for 2 h. Linearized pUC18 (EcoRI) at concentrations ranging from 10 ng to 800 ng was used as template for ligations using T4 DNA ligase. The primer and octamer concentrations used were 12 pmols in each reaction.

Isothermal ligations (37 °C, 45 °C, 55 °C, 65 °C) were also performed with Taq DNA ligase, Ampligase and Pfu DNA ligase using their respective ligation buffers. With T4 DNA ligase, ligation product was detectable at all the isothermal conditions used. At 37 °C, as low as 10 ng of the template produced a detectable ligation product. Larger amounts of template yielded ligation products with intense signal.

None of the other ligases produced any detectable ligation product under isothermal conditions.

## Claims

1. A method for synthesizing a strand of a nucleic acid complementary to at least a portion of a target single stranded nucleic acid template comprising:
a) providing a reaction mixture comprising the template, a primer having at least 15 bases which is complementary to a portion of the target single stranded nucleic acid template and plurality of oligonucleotide 5'-monophosphates each having no more than 10 bases;
b) hybridizing the primer with the template under conditions which permit stable hybridization of the primer but not stable hybridization of the oligonucleotide 5 monophosphates to form a primer-template hybrid having a single stranded region and a double stranded region;
c) ligating to the primer-template hybrid in sequence at least some of the plurality of oligonucleotide 5'-monophosphates in a contiguous manner onto the primer in one continuous process under conditions which permit stable hybridisation of the primer but not stable hybridisation of the oligonucleotide 5'-monophosphates to extend the double stranded region and thereby synthesize a nucleic acid strand which is complementary to the portion of the template, wherein ligation of oligonucleotide 5'-monophosphates only occurs in the presence of hybridised primer.

2. A method according to claim 1 in which the amount of a portion of a double stranded nucleic acid having a first strand and a second strand is amplified, which method comprises:
a) providing in a reaction mixture the double stranded nucleic acid a first primer having at least 15 bases which is complementary to a region of the first strand, a second primer having at least 15 bases which is complementary to a region of the second strand wherein the first and second regions define the portion of the double stranded nucleic acid to be amplified and a plurality of oligonucleotide-5'-monophosphates each consisting of no more than 10 bases;
b) separating the first and second strands of the double stranded nucleic acid;
c) hybridizing the first and second primers with the separated strands; under conditions which permit stable hybridization of the primers but not stable hybridization of the oligonucleotide-5'-monophosphates;
d) ligating onto each of the hybridized first and second primers at least some of the plurality of oligonucleotide 5'-monophosphates in a contiguous manner on one continuous process under conditions which permit stable hybridization of the primers but not stable hybridization of the oligo-nucleotide-5'-monophosphates, to extend the first and second primers, wherein ligation only occurs in the presence of the hybridized primers, thereby producing double stranded nucleic acid; and
e) repeating steps b-d to increase the amount of the portion of double stranded nucleic acid.

3. The method of claim 1 or claim 2 wherein the ligation is performed by means of a ligase enzyme.

4. The method of claim 1 wherein ligation is performed by the T4 DNA ligase.

5. The method of claim 3 wherein the ligation is performed by a ligase enzyme that is thermostable.

6. The method of any preceding claim wherein each of the oligonucleotide 5'-monophosphates contains the same number of bases and the number of bases is from 2 to 6.

7. The method of claim 6 wherein the number of bases is 5.

8. The method of claim 1 or claim 2 wherein each of the oligonucteotide-5'-monophosphates contains the same number of bases and the plurality of oligonucleotide-5'-monophosphates is a set of oligonucleotide-5'-monophosphates preselected to contain only those oligomers necessary to replicate the strand.

9. The method of claim 8 wherein the sequence of the synthesised nucleic acid is known in advance.

10. The method of claim 1 wherein each of the oligonucleotide-5'-monophosphates contains the same number of bases and the plurality of oligonucleotide 5-monophosphates is a library of oligonucleotide 5'-monophosphates comprising at least 50% of all possible oligonucleotide 5'-monophosphates of the same length containing A, C, G and T.

11. The method of claim 1 wherein the synthesis of the strand of nucleic acid is unidirectional and proceeds by ligation to the 3' end of the primer.

12. The method of claim 1 wherein the synthesis of the strand of nucleic acid is unidirectional and proceeds by ligation to the 5' end of the primer.

13. The method of claim 1 or claim 2 wherein the primer or at least one of the primers contains a 5'-phosphate group and the synthesis of the strand or each strand of nucleic acid is bidirectional.

14. The method of claim 1 wherein the primer is immobilized onto a solid phase.

15. The method of claim 14 in which, after step c) the oligonucleotides which are not ligated are removed and the product having a double stranded region is denatured to remove the template and produce an immobilised single stranded nucleic acid product.

16. The method of claim 1 wherein the primer is in a solution.

17. The method of claim 1, 14, 15 or 16 wherein at least some of the oligonucleotide 5'-monophosphates comprise a detectable label.

18. The method of claim 17 wherein all the oligonucleotide-5'-monophosphates are labelled.

19. The method of claim 18 wherein each of the plurality of oligonucleotide 5'- monophosphates contains 5 bases.

20. The method of claim 18 wherein each of the plurality of oligonucleotide 5'-monophosphates is of identical length.

21. The method of claim 18 wherein some of the plurality of oligonucleotide 5'-monophosphates contain a different number of bases from other of the plurality of oligonucteotide 5'-monophosphates.

22. The method of claim 18 wherein the ligation is performed by means of a ligase enzyme.

23. The method of claim 18 wherein the ligase enzyme is selected from T4 ligase, T7 ligase, Tth ligase, Taq ligase and *E. Coli* DNA ligase.

24. The method of claim 18 wherein the ligase enzyme is T4 DNA ligase.

25. The method of claim 18 wherein each labelled oligonucleotide 5'-monophosphate is labelled with the same label.

26. The method of claim 18 wherein each labelled oligonucleotide 5'-monophosphate is labelled with an oligonucleotide 5'-monophosphate-specific label.

27. The method of claim 18 wherein each oligonucleotide 5'-monophosphate contains 1 label.

28. The method of claim 17 or claim 18 wherein the detectable label is selected from radioisotopes, chemiluminescent labels, fluorescent labels, colorimetric labels, enzymes, binding proteins, antigens, antibodies and haptens.

29. The method of claim 18 wherein each label is an oligonucleotide.

30. The method of claim 17 wherein the detectable label is cleaved from the synthesized complementary nucleic acid strand before detection.

31. The method of claim 30 wherein the detection of the cleaved labels is performed by determining the molecular mass of the cleaved labels.

32. The method of any of claims 1 and 18, further comprising the incorporation of at least one nonextendable oligomer, which terminates synthesis of the nucleic acid strand.

33. The method of claim 32 wherein each of the nonextendable oligomers is selected from oligomers which have a dideoxy base at the 3'-end, oligomers which have a blocked 3'-OH group at the 3'-end and oligomers which lack a phosphate group at the 5'-end.

34. The method of claim 18 wherein the synthesis of the nucleic acid strand synthesized in step d) is terminated at a predetermined position by excluding from the plurality of oligonucleotide 5'-monophosphates at least one oligonucleotide 5'-monophosphate which is complementary to the single stranded nucleic acid template and having a sequence necessary to extend the double stranded region.

35. The method of claim 18 wherein the synthesis of the strand of nucleic acid is unidirectional and proceeds by ligation to the 3' end of the primer.

36. The method of claim 18 wherein the primer contains a 5'-phosphate group and the synthesis of the strand of nucleic acid is unidirectional and proceeds by ligation to the 5' end of the primer.

37. The method of claim 18 wherein the primer contains a 5'-phosphate group and the synthesis of the strand of nucleic acid proceeds from both ends of the primer.

38. A method according to any of claims 1, 14, 15 or 16 in which the target single stranded nucleic acid template is an analyte, at least some of the oligonucleotide-5'-monophosphates are labelled and in which, after step c) all non-ligated oligonucleotides are removed and the labelled nucleic acid of step c is detected.

39. The method as claimed in claims 18 or claim 38 wherein the single stranded nucleic acid template has a segment of known sequence, wherein the primer is complementary to a portion of the segment of known sequence and the plurality of labelled oligonucleotide 5'-monophosphates comprises a set of labelled oligonucleotide 5'-monophosphates selected to be complementary to a part of the segment of known sequence of the template adjacent to the primer.

40. The method of claim 38 wherein each of the plurality of oligonucleotide 5'-monophosphates contains 5 bases.

41. The method of claim 38 wherein each of the plurality of oligonucleotide 5'-monophosphates is of identical length.

42. The method of claim 38 wherein some of the plurality of oligonucleotide 5'-monophosphates contain a different number of bases from other of the plurality of oligonucleotide 5'-monophosphates.

43. The method of claim 38 wherein the ligation is performed by means of a ligase enzyme.

44. The method of claim 43 wherein the ligase enzyme is selected from T4 ligase, T7 ligase, Tth ligase, Taq ligase and *E. Coli* DNA ligase.

45. The method of claim 43 wherein the ligase enzyme is T4 DNA ligase.

46. The method of claim 38 wherein each labelled oligonucleotide 5'-monophosphate is labelled with the same label.

47. The method of claim 38 wherein each labelled oligonucleotide 5'-monophosphate is labelled with an oligonucleotide 5'-monophosphate-specific label.

48. The method of claim 38 wherein each oligonucleotide 5'-monophosphate contains 1 label.

49. The method of claim 38 wherein each label is selected from radioisotopes, chemiluminescent labels, fluorescent labels, colorimetric labels and enzymes.

50. The method of claim 38 wherein each label is selected from binding proteins, antigens, antibodies, haptens and oligonucleotides.

51. The method of claim 50 wherein at least one label binds to a branched amplification multimer which is detectably labelled wherein the analyte is detected by detecting the labels on bound amplification multimers.

52. The method of claim 38 further comprising:
a) binding the labels with a conjugate of an enzyme and a substance having specific binding affinity to the labels; and
b) reacting the bound enzyme conjugates with a substrate for the enzyme to produce a detectable reaction product.

53. The method of claim 50 wherein the reaction of the enzyme with the substrate produces chemiluminescence as a detectable product.

54. A method according to claim 38 wherein the label is a fluorescent intercalating dye and in which fluorescence from the intercalated bound label is detected in the detection step.

55. A method according to claim 2 for synthesizing a labelled double stranded nucleic acid wherein both strands are labelled wherein the plurality of oligonucleotide 5'-monophosphates are labelled.

56. A method according to claim 1 for distinguishing among different nucleic acid sequences in a sample wherein
the sample comprises at least one portion of a target single stranded nucleic acid template;
the reaction mixture contains a primer which is complementary to a portion of each of the different nucleic acid sequences and for each sequence to be distinguished, a set of oligonucleotide -5'-monophosphates complementary to the sequence wherein at least one of the oligonucleotide-5'-monophosphates in each set is labelled with a label unique to each set;
the product of step (c) is a labelled complementary nucleic acid strand which contains a label that is specific for and identifies that sequence; and
the method further comprises the additional steps as follows:
d) removing all non ligated oligonucleotide 5'-monophosphates; and
e) detecting the labelled nucleic acid product of step(c) as indicative of the presence of the different nucleic acids in the sample, thereby distinguishing among the different nucleic acids in the sample.

57. The method of claim 56 wherein one of the different nucleic acid sequences represents a normal sequence and the other sequences each contain a different mutation.

58. The method of claim 56 wherein each different nucleic acid sequence contains a different mutation.

59. The method of claim 56 wherein the different sequences represent different genotypes.

60. The method of claim 56 wherein the detection of labelled nucleic acid products allows the differentiation of a heterozygote and first and second homozygotes for a genetic condition.

61. The method of claim 56 wherein the nucleic acids in the sample, if present in double stranded form, are separated into single strands before or while hybridizing the primer.

## Patentansprüche

1. Verfahren zum Synthetisieren eines Stranges einer Nukleinsäure, der zu zumindest einem Teil einer einsträngigen Zielnukleinsäure-Matrize komplementär ist, das folgendes umfasst:
(a) Bereitstellen eines Reaktionsgemisches, das die Matrize, einen Primer mit zumindest 15 Basen, der zu einem Teil der einsträngigen Zielnukleinsäure-Matrize komplementär ist und eine Vielzahl von Oligonukleotid-5'-Monophosphaten, die jeweils nicht mehr als 10 Basen aufweisen, umfasst;
(b) Hybridisieren des Primers mit der Matrize unter Bedingungen, die eine stabile Hybridisierung des Primers, jedoch nicht eine stabile Hybridisierung der Oligonukleotid-5'-Monophosphate erlauben, um ein Primer-Matrizen-Hybrid mit einer einsträngigen Region und einer doppelsträngigen Region zu bilden;
(c) Ligieren an das Primer-Matrizen-Hybrid der Reihe nach zumindest einige der Vielzahl von Oligonukleotid-5'-Monophosphaten in einer zusammenhängenden Weise an den Primer in einem kontinuierlichen Verfahren unter Bedingungen, die eine stabile Hybridisierung des Primers, jedoch nicht eine stabile Hybridisierung der Oligonukleotid-5'-Monophosphate erlauben, um die doppelsträngige Region zu verlängern und dadurch einen Nukleinsäurestrang zu synthetisieren, der dem Teil der Matrize komplementär ist, wobei die Ligation der Oligonukleotid-5'-Monophosphate nur in Gegenwart eines hybridisierten Primers eintritt.

2. Verfahren nach Anspruch 1, bei dem die Menge des Teils einer doppelsträngigen Nukleinsäure mit einem ersten Strang und einem zweiten Strang amplifiziert wird, wobei das Verfahren folgendes umfasst:
(a) Bereitstellen der doppelsträngigen Nukleinsäure, eines ersten Primers mit zumindest 15 Basen, der einem Bereich des ersten Stranges komplementär ist, eines zweiten Primers mit zumindest 15 Basen, der einer Region des zweiten Stranges komplementär ist, wobei die ersten und zweiten Regionen den Teil der doppelsträngigen Nukleinsäure definieren, der amplifiziert werden soll, und einer Vielzahl von Oligonukleotid-5'-Monophosphaten, die jeweils aus nicht mehr als 10 Basen bestehen, in einem Reaktionsgemisch;
(b) Trennen der ersten und zweiten Stränge der doppelsträngigen Nukleinsäure;
(c) Hybridisieren der ersten und zweiten Primer mit den getrennten Strängen; unter Bedingungen, die eine stabile Hybridisierung der Primer, jedoch nicht eine stabile Hybridisierung der Oligonukleotid-5'-Monophosphate ermöglichen;
(d) Ligieren an jeden der hybridisierten ersten und zweiten Primer zumindest einige der Vielzahl von Oligonukleotid-5'-Monophosphaten in einer zusammenhängenden Weise in einem kontinuierlichen Verfahren unter Bedingungen, die eine stabile Hybridisierung der Primer, jedoch nicht eine stabile Hybridisierung der Oligonukleotid-5'-Monophosphate ermöglichen, um die ersten und zweiten Primer zu verlängern, wobei die Ligation nur in Gegenwart der hybridisierten Primer eintritt, wodurch eine doppelsträngige Nukleinsäure erzeugt wird; und
(e) Wiederholen der Schritte (b) bis (d), um die Menge des Teils der doppelsträngigen Nukleinsäure zu erhöhen.

3. Verfahren nach Anspruch 1 oder Anspruch 2, wobei die Ligation mittels eines Ligase-Enzyms durchgeführt wird.

4. Verfahren nach Anspruch 1, wobei die Ligation durch die T4 DNA Ligase durchgeführt wird.

5. Verfahren nach Anspruch 3, wobei die Ligation durch ein Ligase-Enzym durchgeführt wird, das thermostabil ist.

6. Verfahren nach einem der vorhergehenden Ansprüche, wobei jedes der Oligonukleotid-5'-Monophosphate dieselbe Anzahl an Basen enthält und die Anzahl an Basen von 2-6 ist.

7. Verfahren nach Anspruch 6, wobei die Anzahl der Basen 5 ist.

8. Verfahren nach Anspruch 1 oder Anspruch 2, wobei jedes der Oligonukleotid-5'-Monophosphate dieselbe Anzahl von Basen enthält und die Vielzahl von Oligonukleotid-5'-Monophosphaten ein Satz Oligonukleotid-5'-Monophosphate ist, der so vorgewählt ist, dass er nur solche Oligomere enthält, die zum Replizieren des Stranges notwendig sind.

9. Verfahren nach Anspruch 8, wobei die Sequenz der synthetisierten Nukleinsäure im voraus bekannt ist.

10. Verfahren nach Anspruch 1, wobei die Oligonukleotid-5'-Monophosphate dieselbe Anzahl von Basen enthalten und die Vielzahl von Oligonukleotid-5'-Monophosphaten eine Bibliothek aus Oligonukleotid-5'-Monophosphaten ist, die zumindest 50% aller möglichen Oligonukleotid-5'-Monophosphate derselben Länge umfassen, die A, C, G und T enthalten.

11. Verfahren nach Anspruch 1, wobei die Synthese des Stranges der Nukleinsäure unidirektional erfolgt und durch Ligation an das 3' Ende der Primers fortschreitet.

12. Verfahren nach Anspruch 1, wobei die Synthese des Stranges der Nukleinsäure unidirektional erfolgt und durch Ligation an das 5' Ende des Primers fortschreitet.

13. Verfahren nach Anspruch 1 oder Anspruch 2, wobei der Primer oder zumindest einer der Primer eine 5' Phosphat-Gruppe enthält und die Synthese des Stranges oder jedes Nukleinsäurestranges bidirektional erfolgt.

14. Verfahren nach Anspruch 1, wobei der Primer an der festen Phase immobilisiert ist.

15. Verfahren nach Anspruch 14, bei dem nach Schritt (c) die Oligonukleotide, die nicht ligiert sind, entfernt werden und das Produkt, das einen doppelsträngigen Bereich aufweist, zur Entfernung der Matrize und zur Erzeugung eines immobilisierten einsträngigen Nukleinsäureproduktes denaturiert wird.

16. Verfahren nach Anspruch 1, wobei der Primer in einer Lösung vorliegt.

17. Verfahren nach Anspruch 1, 14, 15 oder 16, wobei zumindest einige der Oligonukleotid-5'-Monophosphate eine nachweisbare Markierung aufweisen.

18. Verfahren nach Anspruch 17, wobei alle Oligonukleotid-5'-Monophosphate markiert sind.

19. Verfahren nach Anspruch 18, wobei jedes der Vielzahl von Oligonukleotid-5'-Monophosphaten 5 Basen enthält.

20. Verfahren nach Anspruch 18, wobei jedes der Vielzahl von Oligonukleotid-5'-Monophosphaten eine identische Länge aufweist.

21. Verfahren nach Anspruch 18, wobei zumindest einige der Vielzahl von Oligonukleotid 5'-Monophosphaten eine unterschiedliche Anzahl an Basen als andere der Vielzahl von Oligonukleotid-5'-Monophosphaten enthalten.

22. Verfahren nach Anspruch 18, wobei die Ligation mittels eines Ligase-Enzyms durchgeführt wird.

23. Verfahren nach Anspruch 18, wobei das Ligase-Enzym aus T4 Ligase, T7 Ligase, Tth Ligase, Taq Ligase und E. coli DNA Ligase ausgewählt ist.

24. Verfahren nach Anspruch 18, wobei das Ligase-Enzym T4 DNA Ligase ist.

25. Verfahren nach Anspruch 18, wobei jedes markierte Oligonukleotid-5'-Monophosphat mit derselben Markierung markiert ist.

26. Verfahren nach Anspruch 18, wobei jedes markierte Oligonukleotid-5'-Monophosphat mit einem Oligonukleotid-5'-Monophosphat spezifischen Marker markiert ist.

27. Verfahren nach Anspruch 18, wobei jedes Oligonukleotid-5-Monophosphat einen Marker enthält.

28. Verfahren nach Anspruch 17 oder Anspruch 18, wobei der nachweisbare Marker aus Radioisotopen, chemilumineszierenden Markern, fluoreszierenden Markem, colorimetrischen Markern, Enzymen, Bindungsproteinen, Antigenen, Antikörpern und Haptenen ausgewählt ist.

29. Verfahren nach Anspruch 18, wobei jeder Marker ein Oligonukleotid ist.

30. Verfahren nach Anspruch 17, wobei der nachweisbare Marker aus dem synthetisierten komplementären Nukleinsäurestrang vor dem Nachweis abgespalten wird.

31. Verfahren nach Anspruch 30, wobei der Nachweis der gespaltenen Marker durch Bestimmung der Molekülmasse der abgespaltenen Marker durchgeführt wird.

32. Verfahren nach einem der Ansprüche 1 und 18, das weiterhin den Einbau zumindest eines nicht verlängerbaren Oligomers umfasst, das die Synthese des Nukleinsäurestranges terminiert.

33. Verfahren nach Anspruch 32, wobei jedes der nicht verlängerbaren Oligomere aus Oligomeren, die eine Didesoxybase am 3' Ende aufweisen, aus Oligomeren, die eine blockierte 3'-OH Gruppe am 3' Ende aufweisen und aus Oligomeren ausgewählt ist, denen eine Phosphat Gruppe am 5' Ende fehlt.

34. Verfahren nach Anspruch 18, wobei die Synthese des Nukleinsäurestranges, der in Schritt (d) synthetisiert wird, in einer vorherbestimmen Position durch Ausschließen aus der Vielzahl von Oligonukleotid-5'-Monophosphaten zumindest eines Oligonukleotid-5'-Monophosphates, das zur einsträngigen Nukleinsäure-Matrize komplementär ist und das eine Sequenz aufweist, die zur Verlängerung der doppelsträngigen Region notwendig ist, beendet wird.

35. Verfahren nach Anspruch 18, wobei die Synthese des Stranges der Nukleinsäure unidirektional erfolgt und durch Ligation an das 3' Ende des Primers fortschreitet.

36. Verfahren nach Anspruch 18, wobei der Primer eine 5' Phosphat Gruppe enthält und die Synthese des Stranges der Nukleinsäure unidirektional erfolgt und durch Ligation an das 5' Ende des Primers fortschreitet.

37. Verfahren nach Anspruch 18, wobei der Primer eine 5' Phosphat-Gruppe enthält und die Synthese des Nukleinsäurestranges von beiden Enden des Primers her fortschreitet.

38. Verfahren nach einem der Ansprüche 1, 14, 15 oder 16, bei dem die einsträngige Zielnukleinsäurematrize ein Analyt ist, zumindest einige der Oligonukleotid-5'-Monophosphate markiert sind und bei dem nach Schritt (c) alle nicht ligierten Oligonukleotide entfernt werden und die markierte Nukleinsäure von Schritt (c) nachgewiesen wird.

39. Verfahren nach Anspruch 18 oder Anspruch 38, wobei die einsträngige Nukleinsäurematrize ein Segment bekannter Sequenz aufweist, wobei der Primer zu einem Teil des Segmentes bekannter Sequenz komplementär ist und die Vielzahl markierter Oligonukleotid-5'-Monophosphate einen Satz markierter Oligonukleotid-5'-Monophosphate umfasst, der so ausgewählt ist, dass er zu einem Teil des Segments einer bekannten Sequenz der Matrize, die an einen Primer angrenzt, komplementär ist.

40. Verfahren nach Anspruch 38, wobei jedes der Vielzahl von Oligonukleotid-5'-Monophosphaten 5 Basen enthält.

41. Verfahren nach Anspruch 38, wobei jedes der Vielzahl von Oligonukleotid-5'-Monophosphaten eine identische Länge aufweist.

42. Verfahren nach Anspruch 38, wobei einige der Vielzahl von Oligonukleotid-5'-Monophosphaten eine unterschiedliche Anzahl von Basen als andere aus der Vielzahl von Oligonukleotid-5'-Monophosphaten enthält.

43. Verfahren nach Anspruch 38, wobei die Ligation mittels eines Ligase-Enzyms durchgeführt wird.

44. Verfahren nach Anspruch 43, wobei das Ligase-Enzym aus T4 Ligase, T7 Ligase, Tth Ligase, Taq Ligase und E. coli DNA Ligase ausgewählt ist.

45. Verfahren nach Anspruch 43, wobei das Ligase-Enzym T4 DNA Ligase ist.

46. Verfahren nach Anspruch 38, wobei jedes markierte Oligonukleotid-5'-Monophosphat mit demselben Marker markiert ist.

47. Verfahren nach Anspruch 38, wobei jedes markierte Oligonukleotid-5'-Monophosphat mit einem Oligonukleotid-5'-Monophosphat spezifischen Marker markiert ist.

48. Verfahren nach Anspruch 38, wobei jedes Oligonukleotid-5'-Monophosphat einen Marker enthält.

49. Verfahren nach Anspruch 38, wobei jeder Marker aus Radioisotopen, chemilumineszierenden Markem, fluoreszierenden Markem, colorimetrischen Markem und Enzymen ausgewählt ist.

50. Verfahren nach Anspruch 38, wobei jeder Marker aus Bindungsproteinen, Antigenen, Antikörpern, Haptenen und Oligonukleotiden ausgewählt ist.

51. Verfahren nach Anspruch 50, wobei zumindest ein Marker an ein verzweigtes Amplifikationsmultimer bindet, das nachweisbar markiert ist, wobei der Analyt durch Nachweisen der Marker an gebundenen Amplifikationsmultimeren nachgewiesen wird.

52. Verfahren nach Anspruch 38, das weiterhin folgendes umfasst:
(a) Bindung der Marker mit einem Konjugat eines Enzyms und einer Substanz, die eine spezifische Bindungsaffinität für die Marker aufweist; und
(b) Umsetzen der gebundenen Enzymkonjugate mit einem Substrat für das Enzym, um ein nachweisbares Reaktionsprodukt zu erzeugen.

53. Verfahren nach Anspruch 50, wobei die Reaktion des Enzyms mit dem Substrat eine Chemilumineszenz als nachweisbares Produkt erzeugt.

54. Verfahren nach Anspruch 38, wobei der Marker ein fluoreszierender interkalierender Farbstoff ist und bei dem die Fluoreszenz vom interkalierten, gebundenen Marker im Nachweisschritt nachgewiesen wird.

55. Verfahren nach Anspruch 2 zum Synthetisieren einer markierten doppelsträngigen Nukleinsäure, wobei beide Stränge markiert sind, und wobei die Vielzahl von Oligonukleotid-5'-Monophosphaten markiert ist.

56. Verfahren nach Anspruch 1 zum Unterscheiden zwischen verschiedenen Nukleinsäuresequenzen in einer Probe, wobei die Probe zumindest einen Teil einer einsträngigen Zielnukleinsäurematrize umfasst;
das Reaktionsgemisch einen Primer, der zu einem Teil jeder der unterschiedlichen Nukleinsäuresequenzen und für jede Sequenz, die unterschieden werden soll, komplementär ist, einen Satz Oligonukleotid-5'-Monophosphate, die zur der Sequenz komplementär sind, wobei zumindest eines der Oligonukleotid-5'-Monophosphate in jedem Satz mit einem Marker markiert ist, der für jeden Satz einzigartig ist, enthält; wobei das Produkt von Schritt (c) ein markierter komplementärer Nukleinsäurestrang ist, der einen Marker enthält, der für die Sequenz spezifisch ist und diese identifiziert; und das Verfahren weiterhin die zusätzlichen Schritte wie folgt umfasst:
(d) Entfernen aller nicht ligierten Oligonukleotid-5'-Monophosphate; und
(e) Nachweisen des markierten Nukleinsäureproduktes von Schritt (c) als Hinweis auf das Vorhandensein unterschiedlicher Nukleinsäuren in der Probe, wodurch zwischen den verschiedenen Nukleinsäuren in der Probe unterschieden wird.

57. Verfahren nach Anspruch 56, wobei eine der unterschiedlichen Nukleinsäuresequenzen eine normale Sequenz repräsentiert und die anderen Sequenzen jeweils eine andere Mutation enthalten.

58. Verfahren nach Anspruch 56, wobei jede verschiedene Nukleinsäuresequenz eine andere Mutation enthält.

59. Verfahren nach Anspruch 56, wobei die unterschiedlichen Sequenzen unterschiedliche Genotypen repräsentieren.

60. Verfahren nach Anspruch 56, wobei der Nachweis markierter Nukleinsäureprodukte die Differenzierung einer Heterozygote und einer ersten und zweiten Homozygote für einen genetischen Zustand erlaubt.

61. Verfahren nach Anspruch 56, wobei die Nukleinsäuren in der Probe, wenn sie in doppelsträngiger Form vorliegen, in Einzelstränge aufgeteilt werden, bevor oder während der Primer hybridisiert.

## Revendications

1. Procédé de synthèse d'un brin d'un acide nucléique complémentaire d'au moins une partie d'une matrice d'acide nucléique simple brin cible comprenant :
a) l'apport d'un mélange réactionnel comprenant la matrice, une amorce possédant au moins 15 bases qui est complémentaire d'une partie de la matrice d'acide nucléique simple brin cible et une pluralité d'oligonucléotide 5'-monophosphates ne possédant chacun pas plus de 10 bases ;
b) l'hybridation de l'amorce avec la matrice dans des conditions qui permettent une hybridation stable de l'amorce mais pas une hybridation stable des oligonucléotide 5'-monophosphates pour former un hybride de matrice-amorce possédant une région simple brin et une région double brin
c) la ligature à l'hybride de matrice-amorce en série d'au moins certains oligonucléotide 5'-monophosphates de la pluralité des oligonucléotide 5'-monophosphates d'une manière adjacente sur l'amorce en un procédé continu dans des conditions qui permettent une hybridation stable de l'amorce mais pas une hybridation stable des oligonucléotide 5'-monophosphates, pour étendre la région double brin et de ce fait synthétiser un brin d'acide nucléique qui est complémentaire de la partie de la matrice, procédé dans lequel la ligature des oligonucléotide 5'-monophosphates se produit seulement en présence de l'amorce hybridée.

2. Procédé selon la revendication 1 dans lequel la quantité d'une partie d'un acide nucléique double brin possédant un premier brin et un second brin est amplifiée, le procédé comprenant :
a) l'apport dans un mélange réactionnel de l'acide nucléique double brin, d'une première amorce possédant au moins 15 bases qui est complémentaire d'une région du premier brin, d'une seconde amorce possédant au moins 15 bases qui est complémentaire d'une région du second brin, le premier et le second brins définissant la partie de l'acide nucléique double brin à amplifier, et d'une pluralité d'oligonucléotide 5'-monophosphates ne possédant chacun pas plus de 10 bases ;
b) la séparation des premier brin et second brin de l'acide nucléique double brin ;
c) l'hybridation de la première amorce et de la seconde amorce avec les brins séparés ; dans des conditions qui permettent une hybridation stable des amorces mais pas une hybridation stable des oligonucléotide 5'-monophosphates ;
d) la ligature sur chacune des première amorce et seconde amorce hybridées d'au moins certains oligonucléotide 5'-monophosphates de la pluralité des oligonucléotide 5'-monophosphates d'une manière adjacente en un procédé continu dans des conditions qui permettent une hybridation stable de l'amorce mais pas une hybridation stable des oligonucléotide 5'-monophosphates, pour étendre la première amorce et la seconde amorce, procédé dans lequel la ligature se produit seulement en présence des amorces hybridées, produisant de ce fait un acide nucléique double brin ; et
e) la répétition des étapes b à d pour augmenter la quantité de la partie de l'acide nucléique double brin.

3. Procédé selon la revendication 1 ou la revendication 2 dans lequel la ligature est réalisée au moyen d'une enzyme ligase.

4. Procédé selon la revendication 1 dans lequel la ligature est réalisée par la ligase d'ADN T4.

5. Procédé selon la revendication 3 dans lequel la ligature est réalisée par une enzyme ligase qui est thermostable.

6. Procédé selon l'une quelconque des revendications précédentes dans lequel chacun des oligonucléotide 5'-monophosphates contient le même nombre de bases et le nombre de bases est compris entre 2 et 6.

7. Procédé selon la revendication 6 dans lequel le nombre de bases est 5.

8. Procédé selon la revendication 1 ou la revendication 2 dans lequel chacun des oligonucléotide 5'-monophosphates contient le même nombre de bases et la pluralité des oligonucléotide 5'-monophosphates est un ensemble d'oligonucléotide 5'-monophosphates présélectionné pour contenir seulement ceux des oligomères qui sont nécessaires pour répliquer le brin.

9. Procédé selon la revendication 8 dans lequel la séquence de l'acide nucléique synthétisé est connue à l'avance.

10. Procédé selon la revendication 1 dans lequel chacun des oligonucléotide 5'-monophosphates contient le même nombre de bases et la pluralité des oligonucléotide 5'-monophosphates est une banque d'oligonucléotide 5'-monophosphates contenant au moins 50% de tous les oligonucléotide 5'-monophosphates possibles de même longueur contenant A, C, G et T.

11. Procédé selon la revendication 1 dans lequel la synthèse du brin d'acide nucléique est unidirectionnelle et se produit par ligature à l'extrémité 3' de l'amorce.

12. Procédé selon la revendication 1 dans lequel la synthèse du brin d'acide nucléique est unidirectionnelle et se produit par ligature à l'extrémité 5' de l'amorce.

13. Procédé selon la revendication 1 ou la revendication 2 dans lequel l'amorce ou au moins une des amorces contient un groupe 5'-phosphate et la synthèse du brin ou de chaque brin d'acide nucléique est bidirectionnelle.

14. Procédé selon la revendication 1 dans lequel l'amorce est immobilisée sur une phase solide.

15. Procédé selon la revendication 14 dans lequel, après l'étape c) les oligonucléotides qui ne sont pas ligaturés sont éliminés et le produit possédant une région double brin est dénaturé pour éliminer la matrice et produire un produit acide nucléique simple brin immobilisé.

16. Procédé selon la revendication 1 dans lequel l'amorce est en solution.

17. Procédé selon la revendication 1, 14, 15 ou 16 dans lequel au moins certains des oligonucléotide 5'-monophosphates comprennent un marqueur détectable.

18. Procédé selon la revendication 17 dans lequel tous les oligonucléotide 5'-monophosphates sont marqués.

19. Procédé selon la revendication 18 dans lequel chacun des oligonucléotide 5'-monophosphates de la pluralité des oligonucléotide 5'-monophosphates contient 5 bases.

20. Procédé selon la revendication 18 dans lequel chacun des oligonucléotide 5'-monophosphates de la pluralité des oligonucléotide 5'-monophosphates est de longueur identique.

21. Procédé selon la revendication 18 dans lequel certains oligonucléotide 5'-monophosphates de la pluralité des oligonucléotide 5'-monophosphates contiennent un nombre de bases différent des autres oligonucléotide 5'-monophosphates de la pluralité des oligonucléotide 5'-monophosphates.

22. Procédé selon la revendication 18 dans lequel la ligature est réalisée au moyen d'une enzyme ligase.

23. Procédé selon la revendication 18 dans lequel l'enzyme ligase est choisie parmi une ligase T4, une ligase T7, une ligase Tth, une ligase Taq et une ligase d'ADN de *E. Coli*.

24. Procédé selon la revendication 18 dans lequel l'enzyme ligase est une ligase d'ADN T4.

25. Procédé selon la revendication 18 dans lequel chaque oligonucléotide 5'-monophosphate marqué est marqué avec le même marqueur.

26. Procédé selon la revendication 18 dans lequel chaque oligonucléotide 5'-monophosphate marqué est marqué avec un marqueur spécifique d'oligonucléotide 5'-monophosphate.

27. Procédé selon la revendication 18 dans lequel chaque oligonucléotide 5'-monophosphate contient 1 marqueur.

28. Procédé selon la revendication 17 ou la revendication 18 dans lequel le marqueur détectable est choisi parmi des radio-isotopes, des marqueurs chimioluminescents, des marqueurs fluorescents, des marqueurs colorimétriques, des enzymes, des protéines de liaison, des antigènes, des anticorps et des haptènes.

29. Procédé selon la revendication 18 dans lequel chaque marqueur est un oligonucléotide.

30. Procédé selon la revendication 17 dans lequel, avant détection, le marqueur détectable est clivé à partir du brin d'acide nucléique complémentaire synthétisé.

31. Procédé selon la revendication 30 dans lequel la détection des marqueurs clivés est réalisée en déterminant la masse moléculaire des marqueurs clivés.

32. Procédé selon l'une quelconque des revendications 1 et 18, comprenant de plus l'incorporation d'au moins un oligomère non extensible, qui termine la synthèse du brin d'acide nucléique.

33. Procédé selon la revendication 32 dans lequel chacun des oligomères non extensibles est choisi parmi des oligomères qui possèdent une base didéoxy à l'extrémité 3', des oligomères qui possèdent un groupe 3'-OH bloqué à l'extrémité 3' et des oligomères qui sont dénués de groupe phosphate à l'extrémité 5'.

34. Procédé selon la revendication 18 dans lequel la synthèse du brin d'acide nucléique synthétisé dans l'étape d) est terminée en une position prédéterminée en excluant de la pluralité des oligonucléotide 5'-monophosphates au moins un oligonucléotide 5'-monophosphate qui est complémentaire de la matrice d'acide nucléique simple brin et possédant une séquence nécessaire pour étendre la région double brin.

35. Procédé selon la revendication 18 dans lequel la synthèse du brin d'acide nucléique est unidirectionnelle et se produit par ligature à l'extrémité 3' de l'amorce.

36. Procédé selon la revendication 18 dans lequel l'amorce contient un groupe 5'-phosphate et la synthèse du brin d'acide nucléique est unidirectionnelle et se produit par ligature à l'extrémité 5' de l'amorce.

37. Procédé selon la revendication 18 dans lequel l'amorce contient un groupe 5'-phosphate et la synthèse du brin d'acide nucléique se produit à partir des deux extrémités de l'amorce.

38. Procédé selon l'une quelconque des revendications 1, 14, 15 ou 16 dans lequel la matrice d'acide nucléique simple brin cible est un analyte, au moins certains des oligonucléotide 5'-monophosphates sont marqués et dans lequel, après l'étape c) tous les oligonucléotides non ligaturés sont éliminés et l'acide nucléique marqué de l'étape c) est détecté.

39. Procédé tel que revendiqué dans la revendication 18 ou la revendication 38 dans lequel la matrice d'acide nucléique simple brin possède un segment de séquence connue, dans lequel l'amorce est complémentaire d'une partie du segment de séquence connue et la pluralité des oligonucléotide 5'-monophosphates marqués comprend un ensemble d'oligonucléotide 5'-monophosphates marqués choisi pour être complémentaire d'une partie du segment de séquence connue de la matrice adjacente à l'amorce.

40. Procédé selon la revendication 38 dans lequel chacun des oligonucléotide 5'-monophosphates de la pluralité des oligonucléotide 5'-monophosphates contient 5 bases.

41. Procédé selon la revendication 38 dans lequel chacun des oligonucléotide 5'-monophosphates de la pluralité des oligonucléotide 5'-monophosphates est de longueur identique.

42. Procédé selon la revendication 38 dans lequel certains oligonucléotide 5'-monophosphates de la pluralité des oligonucléotide 5'-monophosphates contiennent un nombre de bases différent des autres oligonucléotide 5'-monophosphates de la pluralité des oligonucléotide 5'-monophosphates.

43. Procédé selon la revendication 38 dans lequel la ligature est réalisée au moyen d'une enzyme ligase.

44. Procédé selon la revendication 43 dans lequel l'enzyme ligase est choisie parmi une ligase T4, une ligase T7, une ligase Tth, une ligase Taq et une ligase d'ADN de *E. Coli*.

45. Procédé selon la revendication 43 dans lequel l'enzyme ligase est une ligase d'ADN T4.

46. Procédé selon la revendication 38 dans lequel chaque oligonucléotide 5'-monophosphate marqué est marqué avec le même marqueur.

47. Procédé selon la revendication 38 dans lequel chaque oligonucléotide 5'-monophosphate marqué est marqué avec un marqueur spécifique d'oligonucléotide 5'-monophosphate.

48. Procédé selon la revendication 38 dans lequel chaque oligonucléotide 5'-monophosphate contient 1 marqueur.

49. Procédé selon la revendication 38 dans lequel chaque marqueur est choisi parmi des radio-isotopes, des marqueurs chimioluminescents, des marqueurs fluorescents, des marqueurs colorimétriques et des enzymes.

50. Procédé selon la revendication 38 dans lequel chaque marqueur est choisi parmi des protéines de liaison, des antigènes, des anticorps, des haptènes et des oligonucléotides.

51. Procédé selon la revendication 50 dans lequel au moins un marqueur se lie à un multimère d'amplification ramifié qui est marqué de manière détectable, dans lequel l'analyte est détecté en détectant les marqueurs sur les multimères d'amplification liés.

52. Procédé selon la revendication 38 comprenant de plus :
a) la liaison des marqueurs avec un conjuguat d'une enzyme et une substance possédant une affinité de liaison spécifique pour les marqueurs ; et
b) la réaction des conjuguats d'enzyme liée avec un substrat de l'enzyme pour produire un produit de réaction détectable.

53. Procédé selon la revendication 50 dans lequel la réaction de l'enzyme avec le substrat produit une chimioluminescence comme produit détectable.

54. Procédé selon la revendication 38 dans lequel le marqueur est un colorant intercalant fluorescent et dans lequel la fluorescence du marqueur lié intercalé est détectée dans l'étape de détection.

55. Procédé selon la revendication 2 pour la synthèse d'un acide nucléique double brin marqué dans lequel les deux brins sont marqués, dans lequel la pluralité des oligonucléotide 5'-monophosphates sont marqués.

56. Procédé selon la revendication 1 pour distinguer parmi différentes séquences d'acide nucléique dans un échantillon, procédé dans lequel
l'échantillon comprend au moins une partie d'une matrice d'acide nucléique simple brin cible ;
le mélange réactionnel contient une amorce qui est complémentaire d'une partie de chacune des séquences d'acide nucléique différentes et pour chaque séquence à distinguer, un ensemble d'oligonucléotide 5'-monophosphates complémentaire de la séquence, au moins un des oligonucléotide 5'-monophosphates dans chaque ensemble étant marqué avec un marqueur unique pour chaque ensemble ;
le produit de l'étape c) est un brin d'acide nucléique complémentaire marqué qui contient un marqueur qui lui est spécifique et qui identifie cette séquence ; et
le procédé comprend de plus les étapes additionnelles suivantes :
d) d'élimination de tous les oligonucléotide 5'-monophosphates non ligaturés ; et
e) de détection du produit acide nucléique marqué de l'étape c) comme indicateur de la présence des différents acides nucléiques dans l'échantillon, distinguant de ce fait parmi les différents acides nucléiques de l'échantillon.

57. Procédé selon la revendication 56 dans lequel une des séquences d'acide nucléique différentes représente une séquence normale et les autres séquences contiennent chacune une mutation différente.

58. Procédé selon la revendication 56 dans lequel chaque séquence d'acide nucléique différente contient une mutation différente.

59. Procédé selon la revendication 56 dans lequel les différentes séquences représentent différents génotypes.

60. Procédé selon la revendication 56 dans lequel la détection des produits acide nucléique marqués permet la différenciation d'un hétérozygote et d'un premier et d'un second homozygotes pour une condition génétique.

61. Procédé selon la revendication 56 dans lequel les acides nucléiques de l'échantillon, s'ils sont présents sous forme double brin, sont séparés en simples brins avant ou pendant l'hybridation de l'amorce.
